(19) Europäisches Patentamt
European Patent Office
Office européen des brevets

(11) Numéro de publication: **0 051 023**
**B1**

# (12) FASCICULE DE BREVET EUROPEEN

(45) Date de publication du fascicule du brevet: **30.05.84**

(51) Int. Cl.³: **C 07 H 15/20**, A 61 K 31/70

(21) Numéro de dépôt: **81401654.9**

(22) Date de dépôt: **21.10.81**

## (54) Nouveaux composés appartenant à la famille des benzoyl- et alpha-hydroxybenzyl-phényl-osides, leur procédé de préparation et leur application en thérapeutique.

(30) Priorité: **29.10.80 FR 8023133**

(43) Date de publication de la demande: **05.05.82 Bulletin 82/18**

(45) Mention de la délivrance du brevet: **30.05.84 Bulletin 84/22**

(84) Etats contractants désignés: **AT BE CH DE FR GB IT LI LU NL SE**

(56) Documents cités:
**FR - A - 2 141 532**
**FR - A - 2 375 248**

**CHEMICAL ABSTRACTS, vol. 78, no. 13, 2 avril 1973, page 7, abrégé 79547a, Columbus, Ohio, US L.W. BROWN et al.:"In vitro and in vivo hydrolysis of 4-benzoyl-phenyl N-methylcarbamate"**
**ARZNEIMITTEL FORSCHUNG, vol. 21, no. 6, (1971) Editeur Cantor K.G. Aulendorf/Württ, DE A.M. DE ROOS et al.:"Investigation into the stability of the ether bond in a series of benzhydryl ethers", pages 818-820**
**"Dictionnaire Français de Médecine et de Biologie", A MANUILA, Vol. III. p. 172, MASSON Editeur, Paris**
**"Biochimie", A LEHNINGER, 6 Ed. pp. 217-239, Flammarion (1972), Paris**

(73) Titulaire: **SOCIETE DE RECHERCHES INDUSTRIELLES S.O.R.I. Société anonyme dite:, 3 rue de Citeaux, F-75012 Paris (FR)**

(72) Inventeur: **Picart, François, 38 rue Devosge, F-21000 Dijon (FR)**

(74) Mandataire: **Clisci, Serge et al, CABINET BEAU DE LOMENIE 55 rue d'Amsterdam, F-75008 Paris (FR)**

**EP 0 051 023 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne, en tant que produits industriels nouveaux, les dérivés de benzoyl-phényl-osides et d'α-hydroxybenzyl-phényl-osides de formule (I) ci-dessous. Elle concerne également leur procédé de préparation et leur application en thérapeutique, notamment en tant qu'agents anti-ulcéreux, anti-agrégants plaquettaires, antithrombotiques, et oxygénateurs cérébraux.

On sait que, dans le passé, on a proposé d'utiliser des phényl-glycosides comme agents doués de propriétés antivirales, voir à cet effet l'article de HITOSHI ARITA, Carbohydrate Research 62, 143–154 (1978).

On connaît par ailleurs d'autres dérivés présentant un groupe benzoyl-phényl lié, d'une part, à un reste polysaccharidique aminé, notamment du produit 19 de l'exemple 1 de la demande FR-A-2 375 248 qui a pour formule développée

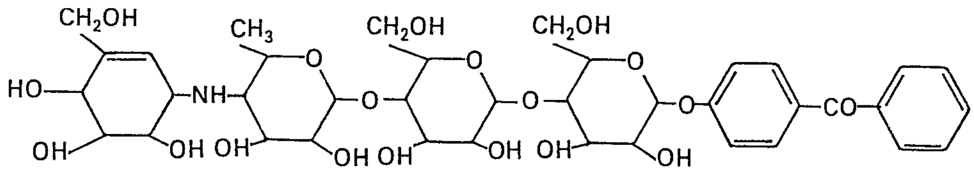

et qui a été préconisée en tant qu'inhibiteur de glucoside-hydrolases dans le traitement en particulier des hyperglycémies de diabétiques, et d'autre part, à un reste acide glucuronique, notamment du résumé des Chemical Abstracts 78, 79547a qui décrit le 4-benzoylphénol glucuronide en tant que métabolite.

On connaît par ailleurs de l'article de A.M. DE ROOS et al. Arzneim.-Forsch. (Drug Res.) 21 (No 6) pages 818–820 (1971), le 4-méthyl-4'-[tétrahydro-2H-pyran-2-yl)oxy]-benzhydrol qui est un composé dans lequel le groupe benzhydrol ou α-hydroxybenzyl-phényle est lié à un radical hétérocyclique n'appartenant pas à la famille des sucres car dépourvu de fonction OH.

On vient de trouver, de façon surprenante, que des composés structurellement différents de ceux de l'art antérieur précité, sont particulièrement intéressants dans le traitement des ulcères et des maladies liées aux troubles circulatoires, et notamment dans le traitement de la sénescence cérébrale.

Les composés selon l'invention sont caractérisés en ce qu'ils sont choisis parmi l'ensemble constitué par

(i) les composés de formule générale

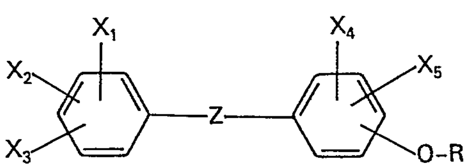

(I)

dans laquelle:

- Z représente CO ou CHOH;
- $X_2$, $X_3$, $X_4$ et $X_5$, identiques ou différents, représentent chacun un atome d'hydrogène, un atome d'halogène, un groupe alkyle en $C_1$–$C_4$, un groupe alkyle en $C_1$–$C_4$ substitué par un ou plusieurs atomes d'halogène (notamment un groupe $CF_3$), un groupe OH, un groupe alkoxy en $C_1$–$C_4$, un groupe alkoxy en $C_1$–$C_4$ substitué par un ou plusieurs atomes d'halogène, un groupe nitro, un groupe cyano, un groupe thiocyano, un groupe isothiocyano, un groupe NR'R'' (où R' et R'', identiques ou différents, représentent chacun l'atome d'hydrogène ou un groupe alkyle en $C_1$–$C_4$);
- $X_1$ représente un atome d'hydrogène, un atome d'halogène, un groupe alkyle en $C_1$–$C_4$, un groupe alkyle en $C_1$–$C_4$ substitué par un ou plusieurs atomes d'halogène, un groupe OH, un groupe alkoxy en $C_1$–$C_4$ un groupe alkoxy en $C_1$–$C_4$ substitué par un ou plusieurs atomes d'halogène, un groupe nitro, un groupe cyano, un groupe thiocyano, un groupe isothiocyano, un groupe NR'R'' (où R' et R'', identiques ou différents, représentent chacun l'atome d'hydrogène ou un groupe alkyle en $C_1$–$C_4$), un groupe $-NH-CS-O-CH_3$ ou un groupe $-O-C(CH_3)_2CO_2-R'''$ (où R''' est un groupe alkyle en $C_1$–$C_4$);
- R représente un radical ose choisi parmi l'ensemble constitué par

a) le radical α-L-rhamnosyle,

b) les radicaux monosaccharides non-hydrolysables, et

c) les radicaux monosaccharides non-hydrolysables où la fonction hydroxyle de l'atome de carbone en position 2 est remplacée par une fonction amine,

les fonctions hydroxyle et amine du groupe R étant susceptibles d'être acétylées, méthylées, benzylées ou sulfatées; et,

(ii) leurs diastéréoisomères quand Z est CHOH.

L'invention comprend également les sels d'addition d'acide des composés de formule (I) lorsque au moins un des groupes $X_1$, $X_2$, $X_3$, $X_4$, $X_5$ et R comporte un groupe basique.

Le groupe –O–R, compte tenu de la structure de la formule (I) donnée ci-dessus, peut être en position ortho, méta ou para par rapport au groupe Z.

Le terme «ose» qui intervient dans la définition du radical R désigne ici toute unité élémentaire glucidique non hydrolysable de formule brute $(CH_2O)_n$, c'est-à-dire les carbohydrates monosaccharidiques non-hydrolysables, d'une part, et l'α-L-rhamnose qui est un dérivé désoxyose de formule brute $C_6H_{12}O_5$. De plus, selon l'invention, l'atome d'hydrogène de chaque fonction hydroxyle du radical ose peut être remplacée par un

groupe $COCH_3$, $CH_3$, $CH_2C_6H_5$ ou sulfate (notamment un groupe $SO_3NH_4$, $SO_3Na$ ou $SO_3K$), et la fonction hydroxyle de l'atome de carbone en position 2 peut être remplacée par une fonction amine, elle-même susceptible d'être substituée par un groupe $COCH_3$, $CH_3$, $CH_2C_6H_5$) ou sulfate (notamment un groupe $SO_3NH_4$, $SO_3Na$ ou $SO_3K$).

Par suite R représente notamment un radical glycosyle tel que β-D-glucosyle, β-D-xylosyle, β-D-galactosyle, β-D-glucosaminyle ou α-L-rhamnosyle, les fonctions hydroxyle et amine pouvant, le cas échéant, être substituées par des groupes acétyle, méthyle, benzyle ou sulfate.

De façon avantageuse, quand $X_1$ représente un groupe $-NH-CS-O-CH_3$ ou $-O-C(CH_3)_2CO_2R'''$, un tel groupe est en position para par rapport au groupe Z et, dans ce cas, chacun des groupes $X_2$, $X_3$, $X_4$ et $X_5$ représente l'atome d'hydrogène.

Par atome d'halogène, on entend ici les atomes de fluor, de chlore, de brome et d'iode, les halogènes préférés étant le fluor, le chlore et le brome, parmi ceux-ci les halogènes les plus intéressants du point de vue thérapeutique sont le chlore et le brome.

Compte tenu des définitions données ci-dessus, l'invention englobe donc les dérivés carbonyle (Z = CO) du type benzoyl-phényl-oside et les dérivés carbinols (Z = CHOH) du type α-hydroxy-benzyl-phényl-oside.

Parmi les composés de formule (I) qui sont préférés selon l'invention, on peut notamment mentionner les dérivés osides où Z est CO ou CHOH; $X_1$, $X_2$, $X_3$, $X_4$ et $X_5$, identiques ou différents, représentent chacun H, Cl, Br, $CH_3$, OH, $OCH_3$, $NO_2$, $NH_2$, $N(CH_3)_2$, NCS, $X_1$ représentant également en position para, par rapport au groupe Z, un groupe $-OC(CH_3)_2CO_2-CH(CH_3)_2$ ou un groupe $-NH-CS-OCH_3$ quand $X_2 = X_3 = X_4 = X_5 = H$; et R représente un radical choisi parmi l'ensemble constitué par les radicaux α-L-rhamnosyle, β-D-glucosyle, β-D-xylosyle, β-D-galactosyle, et β-D-glucosaminyle dans lesquels, le cas échéant, l'atome d'hydrogène des groupes OH peut être remplacé par un reste $COCH_3$, $CH_3$, $CH_2C_6H_5$, $SO_3NH_4$, $SO_3Na$, $SO_3K$, et la fonction amine du groupe β-D-glucosaminyle peut être substituée par un groupe $COCH_3$.

Les composés de formule (I) peuvent être préparés selon une méthode connue en soi selon un mécanisme réactionnel classique. On peut ainsi procéder à l'osidation d'un phényl-phénol de formule:

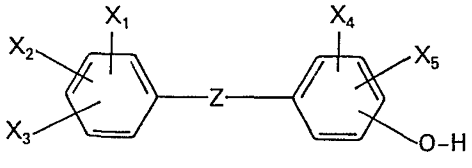

(IIa)

(où Z, $X_1$, $X_2$, $X_3$, $X_4$ et $X_5$ sont définis comme ci-dessus) selon

- la méthode de KOENIG-KNORR [décrite dans l'ouvrage «The Carbohydrates, Chemistry and Biochemistry» (2e édition, Academic Press, New York et Londres 1972) tome IA, pages 295-301] par condensation d'un phénol (IIa) avec un halogénoacétylose en présence d'un catalyseur tel que le cyanure mercurique, $Ag_2O$, $AgCO_3$, $CdCO_3$ ou d'une amine tertiaire telle que la collidine;

- La méthode de HELFERICH (ibidem, pages 292-294) par condensation d'un acétyloside avec un phénol (IIa) en présence d'un acide de Lewis; ou

- la méthode dite à l'ortho-ester (ibidem, pages 300-304). Le procédé préféré que l'on préconise selon l'invention est caractérisé en ce que:

(i) on fait réagir un dérivé de phényl-phénol de formule:

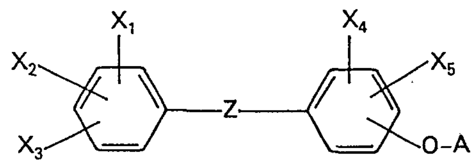

(II)

(où Z, $X_1$, $X_2$, $X_3$, $X_4$ et $X_5$ sont définis comme indiqué ci-dessus et A représente H, Na ou K) avec un dérivé ose choisi parmi l'ensemble constitué par les halogénoacétyloses et les acétyloses dans lesquels le groupe acétylose est un groupe R acétylé tel que défini ci-dessus, dans un solvant inerte, à raison de 1 mole de (II) pour 1,1 à 1,2 mole de dérivé ose;

(ii) et, si nécessaire, on procède à une réaction de désacétylation.

Selon un mode préféré de mise en œuvre du procédé que l'on préconise, on fait réagir au stade (i) 1 mole de dérivé de phénylphénol (II) (où A est Na ou K) avec 1,1 à 1,2 mole d'un halogénoacétylose de formule:

Hal-Ro (III)

[où Hal est F, Cl, Br ou I (de préférence Cl ou Br, pour avoir un rendement optimal), et Ro représente un reste R actétylé], dans un solvant inerte polaire ou apolaire choisi parmi l'ensemble constitué par

a) les diméthylformamide, tétrahydrofuranne, dioxanne, méthanol, éthanol, acétonitrile, nitrométhane, diméthylsulfoxyde, et leurs mélanges, et

b) les mélanges DMF-$CH_2Cl_2$, DMF-$CHCl_3$, DMF-$ClCH_2CH_2Cl$.

De façon avantageuse, on utilisera comme solvant l'acétonitrile. La réaction de (II) avec (III) est réalisée à une température comprise entre 0°C et la température de reflux du milieu réactionnel (avantageusement entre 0°C et 25°C et notamment à la température ambiante) pendant 10 à 40 minutes (de façon avantageuse 10 à 20 minutes pour les composés «carbonyle» Z = CO, et 20 à 30 minutes pour les composés «carbinol» Z = CHOH). Le dérivé obtenu selon cette technique est soumis, le cas échéant, à une réaction de désacétylation.

La réaction de désacétylation du stade (ii) est réalisée par chauffage à reflux dans le méthanol en présence d'un alcoolate métallique de préférence choisi parmi les méthylate de magnésium et méthylate de sodium.

Quand A représente H on recommande d'utiliser pour la réaction du stade (i) un catalyseur tel que mentionné ci-dessus, notamment $Ag_2O$.

Outre ce procédé qui convient pour la synthèse des composés I du type «carbonyle» et du type «carbinol», on peut mettre en œuvre un autre procédé pour l'obtention des composés du type «carbinol» par réduction du dérivé «carbonyle» correspondant. Cette réaction de réduction est mise en œuvre en faisant réagir un composé de formule I où Z = CO avec un agent choisi parmi l'ensemble constitué par les hydrures métalliques $LiAlH_4$ et $KBH_4$, dans un solvant inerte tel que l'éther, le tétrahydrofuranne et les alcools inférieurs notamment le méthanol et l'éthanol, à une température comprise entre 0°C et 50°C (de préférence entre 0°C et 25°C, notamment à la température ambiante).

Les composés de formule I où Z est CHOH peuvent être dédoublés en leurs 2 diastéréoisomères selon une méthode connue en soi, notamment par recristallisation fractionnée. De façon avantageuse, on préconise d'effectuer la résolution du mélange des diastéréoisomères a) par dissolution dudit mélange dans $CH_3OH$-$H_2O$ (1:1) v/v et recristallisation jusqu'à pouvoir rotatoire constant dans $CH_3OH$-$H_2O$ (1:1) v/v, pour obtenir le dextrogyre, puis b) par reprise des filtrats de recristallisation dans $CH_3OH$-$H_2O$ (1:2) v/v et recristallisation jusqu'à pouvoir rotatoire constant dans $CH_3OH$-$H_2O$ (1:2) v/v pour obtenir le lévogyre.

Selon l'invention, on propose une composition thérapeutique caractérisée en ce qu'elle renferme, en association avec un excipient physiologiquement acceptable, au moins un composé choisi parmi l'ensemble constitué par les produits de formule I, leurs diastéréoisomères et leurs sels non toxiques.

Les composés de formule (I) sont utiles en thérapeutique en tant qu'agents anti-ulcéreux, antiagrégants plaquettaires, antithrombotiques ou oxygénateurs cérébraux. Les composés les plus intéressants en thérapeutique sont les produits des exemples 1 (No de code 163), 97 (No de code 265), 98 (No de code 390) et 99 (No de code 391) qui sont particulièrement indiqués en tant qu'agents antithrombotiques veineux, le composé préféré étant le produit de l'exemple 97.

D'autres caractéristiques et avantages de l'invention seront mieux compris à la lecture qui va suivre d'exemples de préparation nullement limitatifs mais donnés à titre d'illustration.

Préparation I

Obtention du [4-(4-nitrobenzoyl)-phényl]-2,3,4-tri (O-acétyl)-β-D-xylopyranoside (No de code 236; exemple 41)

Dans un ballon de 500 ml, on met en suspension le phénate sec obtenu par action de 4,1 g de soude sur 25 g de 4'-paranitrobenzoyl-phénol dans un mélange de 65 ml de DMF et 200 ml de 1,2-dichloroéthane.

On porte le mélange à reflux et on additionne rapidement 45 g de 2,3,4-tri-(O-acétyl)-1-bromo-α-D-xylopyranose. On chauffe ensuite 3 h à reflux. Après hydrolyse, on extrait à l'acétate d'éthyle et on lave la phase organique à la soude à 40 g/l. On évapore à sec et on obtient une huile jaune qui cristallise dans l'éther anhydre. On recristallise enfin dans le méthanol. On obtient ainsi 22 g de produit attendu.

F = 150°C.
$\alpha_D^{20°C} = -33{,}3°$ (c = 0,9 g/l; $ClCH_2CH_2Cl$).

Préparation II

Obtention du [4-(4-nitrobenzoyl)-phényl]-β-D-xylopyranoside (No de code 163; exemple 1)

20 g de produit acétylé obtenu selon la Préparation I sont dissous à chaud 300 ml de méthanol et 2 g de $Mg(OCH_3)_2$. Le mélange est porté à reflux pendant 2 h. On additionne ensuite 1 litre de méthanol et on réchauffe jusqu'à dissolution complète. On filtre la solution jaune obtenue. Après évaporation du solvant, on obtient le produit attendu avec un rendement de 90%.

F = 200°C.
$\alpha_D^{20°C} = -26{,}6°$ (c = 0,6 g/l; méthanol).

Préparation III

Obtention du [4-(4-nitrobenzoyl)-phényl]-2-(N-acétyl)-β-D-glucosaminide (No de code 207; exemple 44)

Dans un ballon de 500 ml, on met en suspension le phénate sec, obtenu par action de 0,540 g de NaH sur 5 g de 4'-paranitro-benzoylphénol, dans un mélange de 25 ml de DMF et 25 ml de 1,2-dichloroéthane. On ajoute au milieu réactionnel 8,3 g d'actétochloroglucosamine et on agite 3 h à 40°C. Après hydrolyse, on extrait à l'acétate d'éthyle, lave à la soude à 40 g/l et enfin à l'eau.

Après évaporation du solvant, on obtient une huile qui précipite dans l'éther. Le dérivé acétylé obtenu est recristallisé dans l'acétate d'éthyle. (Rendement = 59%; F = 238°C).

Ce dérivé acétylé est mis en suspension dans 150 ml de méthanol avec 0,150 g de méthylate de sodium. On agite le milieu réactionnel pendant 2 h à température ambiante puis on hydrolyse sur de la glace. Après filtration, lavage à l'eau et recristallisation dans le méthanol, on obtient 4,4 g (rendement = 80%) du produit attendu.

F = 204°C.
$\alpha_D^{20°C} = +12.5°$ (c = 0,6 g/l, méthanol).

Préparation IV

Obtention du [3-(4-trifluorméthylbenzoyl)-phényl]-β-D-xylopyranoside (No de code 428; exemple 53)

Dans un ballon de 250 ml, on additionne dans l'ordre: 9,4 g de 3-(paratrifluorométhylbenzoyl)-phénol, 15 g d'acétobromoxylose, la quantité

d'oxyde d'argent sec (fraîchement préparé par action de la soude à 40 g/l sur 14 g de nitrate d'argent) et 100 ml d'acétonitrile. On agite 10–20 min sous atmosphère d'azote et à l'abri de la lumière.

Après filtration puis hydrolyse, on extrait à l'acétate d'éthyle, lave à la soude à 40 g/l puis à l'eau. Après évaporation du solvant, on recristallise dans le mélange méthanol-eau (1:1) v/v. On obtient ainsi 10 g (rendement = 55%) du dérivé acétylé (F = 90°C) que l'on reprend dans 100 ml de méthanol avec 0,1 g de méthylate de sodium. On agite pendant 1 h puis passe sur résine Amberlite IR 120 H, filtre puis évapore le solvant. Après recristallisation dans le méthanol, on obtient 5 g (rendement = 65%) du produit attendu.

F = 120°C.
$\alpha_D^{20°C}$ = −38° (c = 0,5 g/l; méthanol).

Préparation V
Obtention de [4-(4-chlorobenzoyl)-phényl]-3,4,6-tri-(ammoniumsulfate)-2-N-acétyl-β-D-glucosaminide (No de code 358; exemple 67)

Sous atmosphère d'azote et à −10°C, on mélange 15 g de [4-(parachlorobenzoyl)-phényl]-2-N-acétyl-β-D-glucosaminide, 29,6 ml de pyridine et 150 ml de DMF. On ajoute goutte à goutte 12,3 ml de chlorure de sulfonyle. Après agitation du milieu réactionnel à température ambiante (15–25°C) pendant 12 h, on amène la solution à pH 9 par addition de bicarbonate de sodium. Après extraction à l'acétate d'éhyle, on évapore la phase aqueuse à 35°C maximum. On reprend alors par 400 ml d'eau et on filtre. Le filtrat est passé trois fois sur résine OC1031 (résine neutre styrénique permettant de réaliser des séparations par liaison hydrogène et commercialisée par la Société BAYER sous la désignation commerciale de LEWATIT). On évapore ensuite les phases alcooliques. Le résidu est repris dans un mélange butanol-éthanol-eau-ammoniaque (40:12:10:1) v/v et la solution passée sur colonne d'alumine neutre. Après évaporation du solvant, on obtient 16,5 g du produit attendu.

F = 200°C (avec décomposition).
$\alpha_D^{20°C}$ = −2,1° (c = 2,6 g/l; eau).

Préparation VI
Obtention du [4(4-nitro-α-hydroxybenzyl)-phényl]-β-D-xylopyranoside (No de code: 265; exemple 97)

On met en suspension 10 g (26,6 millimoles) de [4-(4-nitrobenzoyl)-phényl]-β-D-xylopyranoside (No de code 163; exemple 1; cf. Préparation II) dans 200 ml de méthanol puis on ajoute 1,56 g (26,6 millimoles) de $KBH_4$. Le milieu réactionnel ainsi obtenu est agité pendant 2 h la température ambiante (15–25°C). – Le déroulement de la réaction de réduction est contrôlé par CCM [solvant: toluène – méthanol (3:1) v/v] –. Après évaporation sous vide du méthanol, on reprend à l'acétate d'éthyle puis lave à l'eau (3 × 50 ml). On sèche sur sulfate de magnésium puis après évaporation du solvant on recristallise dans le mélange méthanol – eau (3:7) v/v. On obtient 6,5 g (rendement = 65%) de produit attendu.

F = 142°C
$\alpha_D^{20°C}$ = −17° (c = 0,5 g/l; méthanol).

Préparation VII
Obtention du [4-(4-nitro-α-hydroxybenzyl)-phényl]-2,3,4-tri-(O-acétyl)-β-D-xylopyranoside (exemple 96)

Dans un ballon protégé de la lumière et muni d'un tube à $CaCl_2$ on met 2,45 g de 4-(4-nitro-α-hydroxy-benzyl)-phénol, 3,4 g d'acétobromoxylose, 2,4 g d'oxyde d'argent fraîchement préparés et 200 ml d'acétonitrile. On agite ½ heure à température ambiante, puis on filtre sur verre fritté. Après hydrolyse du filtrat, on extrait à l'acétate d'éthyle puis lave à l'eau. On sèche la phase organique, filtre puis évapore le solvant. On obtient une huile qui est purifiée sur colonne de silice [éluant: toluène – acétate d'éthyle (4:1) v/v]. On obtient ainsi 2 g (rendement = 40%) de produit attendu.

F = 80°C.
$\alpha_D^{20°C}$ = −25° (c = 0,5 g/l; méthanol).

Préparation VIII

En désacylant le produit qui a été obtenu selon la Préparation VII, par chauffage au reflux dans $CH_3OH$ en présence de méthylate de magnésium, on obtient le [4-(4-nitro-α-hydroxy-benzyl-phényl]-β-D-xylopyranoside (qui fait l'objet de la Préparation VI ci-dessus).

Préparation IX
Séparation des diastéréoisomères dextrogyre (No de code 390; exemple 98) et lévogyre (No de code 391; exemple 99) de [4-(4-nitro-α-hydroxybenzyl)-phényl]-β-D-xylopyranoside.

On dissout 40 g du produit de l'exemple 97 (No de code 265) dans 400 ml du mélange méthanol – eau (1:1) v/v et on recristallise jusqu'à pouvoir rotatoire constant. On obtient 16 g du diastéréoisomère dextrogyre.

F = 162°C.
$\alpha_D^{20°C}$ = +21° (c = 0,48 g/l; méthanol).

Les filtrats des recristallisations précédentes sont repris dans 300 ml du mélange méthanol – eau (1:2) v/v et on recristallise jusqu'à pouvoir rotatoire constant. On obtient 15 g du diastéréoisomère lévogyre.

F = 158°C.
$\alpha_D^{20°C}$ = −50° (c = 0,48 g/l; méthanol).

Préparation X
Obtention du [2-(4-nitrobenzoyl)-phényl]-2,3,4-tri-(O-acétyl)-β-D-xylopyranoside (exemple 141)

Dans un ballon de 100 ml, on introduit 120 mg de NaH et 10 cm³ de DMSO. Après 15 minutes on ajoute 2 × $10^{-3}$ mole (486 mg) de 2′-paranitrobenzylphénal puis 4 × $10^{-3}$ mole (1,3 g) d'acéto-

bromoxylose et 5 ml de DMSO. On agite pendant 1 heure à la température ambiante (15–20°C. On extrait à l'éther et lave à l'eau la phase éthérée. Après évaporation de l'éther on obtient 1,57 g de produit attendu brut que l'on chromatographie sur colonne de silice [éluant: toluène – acétate d'éthyle (4:1) v/v] pour recueillir 400 mg (rendement = 40%) de produit pur attendu.

F = 142°C.

Préparation XI
Obtention du [2-(4-nitrobenzoyl)-phényl]-β-D-xylopyranoside (Exemple 57)

Selon le mode opératoire décrit dans la Préparation II ci-dessus, et à partir de 50 mg de [2-(4-nitrobenzoyl)-phényl]-2,3,4-tri-(O-acétyl)-β-D-xylopyranoside obtenu selon la Préparation X, on obtient le produit attendu avec un rendement de 90%.

F = 164°C.

De façon non limitative, on a consigné dans le Tableau I ci-après (où la position des substituants a été donnée arbitrairement, la numérotation des sommets des noyaux phényle étant effectuée à partir du groupe Z central) un certain nombre de composés de formule (I) selon l'invention. Dans le Tableau II ci-après, on a également consigné les propriétés physiques d'une partie de ces composés, à savoir le point de fusion (F) et le pouvoir rotatoire ($\alpha_D^{20°C}$), pour ce dernier ont été précisées la nature du solvant et la concentration (en g/l).

On a résumé dans les Tableaux III à VII ci-après les résultats des essais (toxicité, activités anti-ulcéreuse, anti-agrégante plaquettaire, anti-thrombotique veineuse et anti-hypoxique) entrepris sur un certain nombre de produits selon l'invention. Les protocoles opératoires qui ont été utilisés sont les suivants.

Toxicité aiguë

La toxicité aiguë a été étudiée chez la souris par voie I.P. Elle est exprimé sous forme de DL-50 (dose létale entraînant la mort de la moitié des animaux) ou de DL-0 (dose maximale non létale). Les résultats sont donnés dans le Tableau III.

Agrégation spontanée

La technique utilisée est celle de SANDERS décrite dans Laboratory Animal Science 27 (no 5), pages 757–761 (1977).

Après administration à 100 mg/kg I.P. (sauf précision contraire donnée dans le Tableau V ci-après) du produit à tester à des rats mâles anciens reproducteurs, on effectue deux prélèvements sanguins, l'un sur citrate de sodium et l'autre sur citrate de sodium formolé à l'instant t = 5 h.

Après centrifugation des prélèvements, une numération plaquettaire est réalisée sur le surnageant. L'index d'agrégation spontanée selon WU et HOOK Stroke 6, 521–524 (1975) est exprimé par la relation:

$$I = \frac{\text{Nombre de plaquettes après fixation au formol}}{\text{Nombre de plaquettes circulantes}}$$

Les résultats relatifs au pourcentage de l'inhibition de l'agrégation plaquettaire sont consignés dans le Tableau V.

Thrombose veineuse

La technique utilisée est voisine de celle décrite par PETERSON-ZUCKER dans Thrombo. Diath. Haemorh. 23, 1, (1970). On crée un thrombus occlusif au niveau de la veine cave inférieure de rat par action combinée d'une altération endothéliale et d'une stase veineuse pendant 15 min et 4 à 5 h après administration du produit à tester (100 mg/kg I.P.). Les résultats sont consignés dans le Tableau VI ci-après.

Ulcère à l'aspirine

L'expérimentation est réalisée sur des rats mâles WISTAR de 180 à 200 g. Les produits à tester sont administrés à 100 mg/kg I.P. (sauf indication contraire donnée dans le Tableau IV).

A t = 0, les rats sont mis à jeun et on pratique une première administration du produit à tester.

A t = 18 heures, on administre per os 2 ml d'une suspension ulcérigène à 192 mg d'aspirine/kg puis on pratique une deuxième administration du produit à tester.

A t = 22 heures, les animaux sont sacrifiés et on cote les ulcères:

petits ulcères punctiformes: note 1
ulcères plus étendus: note 3
ulcères très étendus ou très profonds : note 9.

Cette cotation est normalisée par rapport à un lot témoin et à la cimétidine (un produit de référence) à laquelle l'index 1 a été attribué.

Hypoxie

L'expérimentation est réalisée sur des lots de 20 souris SWISS mâles pesant de 20 à 30 g. On administre aux souris chaque produits à tester par voie intrapéritonéale à une dose correspondant au 1/10e de la DL-50 ou à une dose de 100 mg/kg quand la DL-0 est supérieure ou égale à 800 mg/kg. Les souris sont placées dans une atmosphère azote-oxygène (95:5) v/v. On mesure ensuite le temps de survie des souris jusqu'à 15 minutes maximum.

Les résultats consignés dans le Tableau VII sont exprimés en pourcentage de survie par rapport à un lot témoin non traité et un lot traité par un produit de référence anti-hypoxique [spécialité connue sous le nom de marque de «DUXIL» et qui est constituée par un mélange d'Almitrine bis-méthanesulfonate et de Raubasine dans le rapport pondéral 3:1], le produit de référence anti-hypoxique étant administré à la dose de 18 mg/kg I.P.

Les résultats concernant l'activité anti-ulcéreuse des produits de formule I sont consignés dans le Tableau IV ci-après.

Les produits de formule (I) peuvent être administrés notamment par voie orale, sous forme de gélules ou de comprimés dragéifiés ou non renfermant chacun 0,05 à 1 g d'au moins un composé de formule (I) en tant que principe actif, et de préférence 0,1 à 0,5 g, d'une part, et par voie injectable, sous forme de solutions renfermant de 0,05 à 0,3 g de principe actif dans 2 à 10 $cm^3$ d'eau distillée, d'autre part. Ces formes galéniques pourront être administrées à raison de 1 à 3 prises par jour.

Tableau I

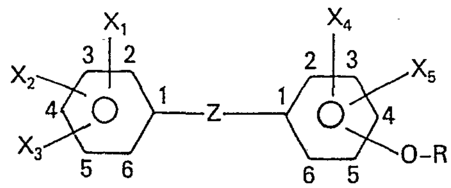

| Ex. | No de code | Z | $X_1$ | $X_2$ | $X_3$ | $X_4$ | $X_5$ | Pos. -O-R | R |
|---|---|---|---|---|---|---|---|---|---|
| 1 | 163 | CO | 4-$NO_2$ | H | H | H | H | para | β-D-Xyl |
| 2 | 459 | CO | 3-$NO_2$ | H | H | H | H | para | β-D-Xyl |
| 3 | 171 | CO | 4-Cl | H | H | H | H | para | β-D-Xyl |
| 4 | 433 | CO | 4-Br | H | H | H | H | para | β-D-Xyl |
| 5 | 456 | CO | 2-Cl | H | H | H | H | para | β-D-Xyl |
| 6 | 511 | CO | 2-$NO_2$ | H | H | H | H | para | β-D-Xyl |
| 7 | 554 | CO | 2-$NH_2$ | H | H | H | H | para | β-D-Xyl |
| 8 | 193 | CO | 4-$NH_2$ | H | H | H | H | para | β-D-Xyl |
| 9 | 466 | CO | 3-$NH_2$ | H | H | H | H | para | β-D-Xyl |
| 10 | 676 | CO | 4-$N(CH_3)_2$ | H | H | H | H | para | β-D-Xyl |
| 11 | 227 | CO | 4-$CF_3$ | H | H | H | H | para | β-D-Xyl |
| 12 | 229 | CO | 4-$CH_3$ | H | H | H | H | para | β-D-Xyl |
| 13 | 465 | CO | 3-$CF_3$ | H | H | H | H | para | β-D-Xyl |
| 14 | 435 | CO | 2-$CH_3$ | H | H | H | H | para | β-D-Xyl |
| 15 | - | CO | 2-$OCH_3$ | H | H | H | H | para | β-D-Xyl |
| 16 | 476 | CO | 3-$OCH_3$ | H | H | H | H | para | β-D-Xyl |
| 17 | 486 | CO | 4-$OCH_3$ | H | H | H | H | para | β-D-Xyl |
| 18 | 262 | CO | H | H | H | H | H | para | β-D-Xyl |
| 19 | 228 | CO | 4-$NO_2$ | H | H | H | H | para | β-D-Glu |
| 20 | 264 | CO | 4-$NO_2$ | H | H | H | H | para | β-D-Gal |
| 21 | 243 | CO | 2-$CH_3$ | H | H | 3-$CH_3$ | 5-$CH_3$ | para | β-D-Xyl |
| 22 | 560 | CO | 4-Cl | H | H | 3-$CH_3$ | 5-$CH_3$ | para | β-D-Xyl |
| 23 | 487 | CO | 4-$NO_2$ | H | H | 3-$CH_3$ | 5-$CH_3$ | para | β-D-Xyl |
| 24 | - | CO | 4-$NH_2$ | H | H | 3-$CH_3$ | 5-$CH_3$ | para | β-D-Xyl |
| 25 | 241 | CO | H | H | H | 3-$NO_2$ | 5-$CH_3$ | para | β-D-Xyl |
| 26 | 242 | CO | 2-$CH_3$ | H | H | 3-$CH_3$ | 5-$CH_3$ | para | β-D-Glu-NHAc |
| 27 | 357 | CO | 4-OH | 2$CH_3$ | H | 3-$CH_3$ | 5-$CH_3$ | para | β-D-Glu |
| 28 | 572 | CO | 4-Cl | H | H | 3-$CH_3$ | H | para | β-D-Xyl |
| 29 | 457 | CO | 4-$NO_2$ | H | H | 3-$CH_3$ | H | para | β-D-Xyl |
| 30 | - | CO | 4-$NH_2$ | H | H | 3-$CH_3$ | H | para | β-D-Xyl |
| 31 | 431 | CO | 2-Cl | 4-Cl | H | H | H | para | β-D-Xyl |
| 32 | 462 | CO | 2-$CH_3$ | 4-$CH_3$ | 6-$CH_3$ | H | H | para | β-D-Xyl |
| 33 | 510 | CO | 3-$OCH_3$ | 4-$OCH_3$ | 5-$OCH_3$ | H | H | para | β-D-Xyl |
| 34 | 652 | CO | 2-$CH_3$ | 4-$CH_3$ | 6-$CH_3$ | 3-$CH_3$ | 5-$CH_3$ | para | β-D-Xyl |
| 35 | - | CO | 4-$NO_2$ | 2-$CH_3$ | H | 3-$CH_3$ | 5-$CH_3$ | para | β-D-Xyl |
| 36 | - | CO | 4-$NH_2$ | 2-$CH_3$ | H | 3-$CH_3$ | 5-$CH_3$ | para | β-D-Xyl |
| 37 | 397 | CO | 2-$CH_3$ | 6-$CH_3$ | H | 3-$CH_3$ | 5-$CH_3$ | para | β-D-Glu-NHA c |
| 38 | - | CO | 4-$NH_2$ | H | H | H | H | para | β-D-Glu-NHA c |
| 39 | - | CO | 4-$NH_2$ | H | H | H | H | para | β-D-Glu |
| 40 | - | CO | 4-$NH_2$ | H | H | H | H | para | β-D-Gal |
| 41 | 236 | CO | 4-$NO_2$ | H | H | H | H | para | $(OAc)_3$-β-D-Xyl |
| 42 | 260 | CO | 4-NCS | H | H | H | H | para | $(OAc)_3$-β-D-Xyl |
| 43 | 172 | CO | 4-Cl | H | H | H | H | para | β-D-Glu-NHAc |
| 44 | 207 | CO | 4-$NO_2$ | H | H | H | H | para | β-D-Glu-NHAc |
| 45 | 430 | CO | H | H | H | H | H | méta | β-D-Xyl |

Tableau I (suite)

| Ex. | No de code | Z | $X_1$ | $X_2$ | $X_3$ | $X_4$ | $X_5$ | Pos. -O-R | R |
|---|---|---|---|---|---|---|---|---|---|
| 46 | 650 | CO | 4-$NO_2$ | H | H | H | H | méta | β-D-Xyl |
| 47 | – | CO | 4-$NH_2$ | H | H | H | H | méta | β-D-Xyl |
| 48 | 427 | CO | 4-Cl | H | H | H | H | méta | β-D-Xyl |
| 49 | 432 | CO | 4-$CH_3$ | H | H | H | H | méta | β-D-Xyl |
| 50 | – | CO | 4-$CH_3$ | H | H | 4-$CH_3$ | H | méta | β-D-Xyl |
| 51 | – | CO | 3-$CF_3$ | H | H | H | H | méta | β-D-Xyl |
| 52 | 434 | CO | 2-$CH_3$ | H | H | H | H | méta | β-D-Xyl |
| 53 | 428 | CO | 4-$CF_3$ | H | H | H | H | méta | β-D-Xyl |
| 54 | 429 | CO | 4-$OCH_3$ | H | H | H | H | méta | β-D-Xyl |
| 55 | – | CO | H | H | H | H | H | ortho | β-D-Xyl |
| 56 | – | CO | 4-Cl | H | H | H | H | ortho | β-D-Xyl |
| 57 | – | CO | 4-$NO_2$ | H | H | H | H | ortho | β-D-Xyl |
| 58 | – | CO | 4-$NH_2$ | H | H | H | H | ortho | β-D-Xyl |
| 59 | – | CO | 2-$CH_3$ | H | H | 5-Cl | H | ortho | β-D-Xyl |
| 60 | 677 | CO | 2-$CH_3$ | H | H | 5-$CH_3$ | H | ortho | β-D-Xyl |
| 61 | – | CO | 4-$NO_2$ | H | H | 5-$CH_3$ | H | ortho | β-D-Xyl |
| 62 | – | CO | 4-$NH_2$ | H | H | 5-$CH_3$ | H | ortho | β-D-Xyl |
| 63 | – | CO | 4-$NO_2$ | H | H | 5-Cl | H | ortho | β-D-Xyl |
| 64 | – | CO | 4-$NH_2$ | H | H | 5-Cl | H | ortho | β-D-Xyl |
| 65 | 222 | CO | 4-NHC-<br>$OCH_3$<br>‖<br>S | H | H | H | H | para | β-D-Xyl |
| 66 | 289 | CO | $CH_3$<br>│<br>4-OC-$CO_2$-$CH(CH_3)_2$<br>│<br>$CH_3$ | H | H | H | H | para | β-D-Xyl |
| 67 | 358 | CO | 4-Cl | H | H | H | H | para | $(SO_3NH_4)_3$-β-D-Glu-NHAc |
| 68 | 416 | CO | 2-$CH_3$ | H | H | 3-$CH_3$ | 5-$CH_3$ | para | $(SO_3NH_4)_3$-β-D-Glu-NHAc |
| 69 | 488 | CO | 2-$CH_3$ | H | H | H | H | para | $(OAc)_3$-β-D-Xyl |
| 70 | 490 | CO | 2-Cl | H | H | H | H | para | $(OAc)_3$-β-D-Xyl |
| 71 | 497 | CO | 2-$CH_3$ | 4-$CH_3$ | 6-$CH_3$ | H | H | méta | β-D-Xyl |
| 72 | 498 | CO | 3-$NO_2$ | H | H | H | H | para | $(OSO_3Na)_3$-β-D-Xyl, 3 $H_2O$ |
| 73 | 499 | CO | 2-$CH_3$ | 4-$CH_3$ | 6-$CH_3$ | H | H | para | $(OAc)_3$-β-D-Xyl |
| 74 | 500 | CO | 2-Cl | 4-Cl | H | H | H | para | $(OAc)_3$-β-D-Xyl |
| 75 | 501 | CO | 4-Br | H | H | H | H | para | $(OAc)_3$-β-D-Xyl |
| 76 | 502 | CO | 4-$CH_3$ | H | H | H | H | para | $(OAc)_3$-β-D-Xyl |
| 77 | 503 | CO | 4-$NH_2$ | H | H | H | H | para | $(OAc)_3$-β-D-Xyl |
| 78 | 518 | CO | 2-$NO_2$ | H | H | H | H | para | $(OAc)_3$-β-D-Xyl |
| 79 | 519 | CO | 3-$OCH_3$ | 4-$OCH_3$ | 5-$OCH_3$ | H | H | para | $(OAc)_3$-β-D-Xyl |
| 80 | 520 | CO | 3-$NO_2$ | H | H | H | H | para | $(OAc)_3$-β-D-Xyl |
| 81 | 521 | CO | 4-$OCH_3$ | H | H | H | H | para | $(OAc)_3$-β-D-Xyl |
| 82 | 522 | CO | 4-Cl | H | H | H | H | méta | $(OAc)_3$-β-D-Xyl |
| 83 | 523 | CO | H | H | H | H | H | méta | $(OAc)_3$-β-D-Xyl |
| 84 | 525 | CO | 3-$OCH_3$ | 4-OH | 5-$OCH_3$ | H | H | para | $(OAc)_3$-β-D-Xyl |
| 85 | 477 | CO | 2-$CH_3$ | H | H | 3-$CH_3$ | H | para | β-D-Xyl |
| 86 | 512 | CHOH | 2-Cl | 4-Cl | H | H | H | para | β-D-Xyl |
| 87 | 513 | CHOH | 3-$NO_2$ | H | H | H | H | para | β-D-Xyl |
| 88 | 514 | CHOH | 2-Cl | H | H | H | H | para | β-D-Xyl |
| 89 | 515 | CHOH | 2-$CH_3$ | H | H | H | H | para | β-D-Xyl |
| 90 | 516 | CHOH | 4-$CH_3$ | H | H | H | H | para | β-D-Xyl |
| 91 | 517 | CHOH | 4-Cl | H | H | H | H | para | β-D-Xyl |
| 92 | 526 | CHOH | H | H | H | H | H | para | β-D-Xyl |
| 93 | 527 | CHOH | 3-$CF_3$ | H | H | H | H | para | β-D-Xyl |
| 94 | 528 | CHOH | 4-Cl | H | H | H | H | para | β-D-Xyl |
| 95 | 529 | CHOH | 4-Br | H | H | H | H | para | β-D-Xyl |
| 96 | 559 | CHOH | 4-$NO_2$ | H | H | H | H | para | $(OAc)_3$-β-D-Xyl |
| 97[a] | 265 | CHOH | 4-$NO_2$ | H | H | H | H | para | β-D-Xyl |
| 98[b] | 390 | CHOH | 4-$NO_2$ | H | H | H | H | para | β-D-Xyl |

Tableau I (suite)

| Ex. | No de code | Z | $X_1$ | $X_2$ | $X_3$ | $X_4$ | $X_5$ | Pos. -O-R | R |
|---|---|---|---|---|---|---|---|---|---|
| 99[c] | 391 | CHOH | 4-$NO_2$ | H | H | H | H | para | β-D-Xyl |
| 100 | 411 | CHOH | 4-Cl | H | H | H | H | para | β-D-Glu-NHAc |
| 101 | 555 | CHOH | 2-$NO_2$ | H | H | H | H | para | β-D-Xyl |
| 102 | 551 | CO | 2-Cl | H | H | 3-$CH_3$ | 5-$CH_3$ | para | β-D-Xyl |
| 103 | 550 | CO | 2-$CH_3$ | H | H | 3-$CH_3$ | H | para | $(OAc)_3$-β-D-Xyl |
| 104 | 549 | CO | 4-$NO_2$ | H | H | 3-$CH_3$ | H | para | $(OAc)_3$-β-D-Xyl |
| 105 | 558 | CHOH | 2-$CH_3$ | H | H | 3-$CH_3$ | H | para | β-D-Xyl |
| 106[d] | 557 | CHOH | 4-$CH_3$ | H | H | H | H | méta | β-D-Xyl |
| 107[d] | 556 | CHOH | 4-$CH_3$ | H | H | H | H | méta | β-D-Xyl |
| 108 | 541 | CO | 3-$CF_3$ | H | H | H | H | para | $(OAc)_3$-β-D-Xyl |
| 109 | 561 | CO | 4-$NO_2$ | H | H | H | H | para | $(OAc)_3$-β-D-Glu-NHAc |
| 110 | 562 | CO | 4-$NO_2$ | H | H | H | H | para | $(OAc)_4$-β-D-Glu |
| 111 | 563 | CO | 4-$CH_3$ | H | H | H | H | para | β-D-Glu-NHAc |
| 112 | 564 | CO | 4-$CH_3$ | H | H | H | H | para | $(OAc)_3$β-D-Glu-NHAc |
| 113 | 565 | CO | 2-Cl | H | H | 3-$CH_3$ | 5-$CH_3$ | para | $(OAc)_3$-β-D-Xyl |
| 114 | 566 | CO | 4-Cl | H | H | 3-$CH_3$ | 5-$CH_3$ | para | $(OAc)_3$-β-D-Xyl |
| 115 | 662 | CHOH | 4-$NO_2$ | H | H | H | H | para | β-D-Gal |
| 116 | 568 | CO | 4-$N(CH_3)_2$ | H | H | H | H | para | $(OAc)_3$-β-D-Xyl |
| 117 | 570 | CHOH | H | H | H | H | H | méta | β-D-Xyl |
| 118 | 573 | CO | 4-$CF_3$ | H | H | H | H | para | β-D-Glu-NHAc |
| 119 | 574 | CO | 4-$NO_2$ | H | H | 3-$CH_3$ | 5-$CH_3$ | para | β-D-Glu-NHAc |
| 120 | 575 | CO | 2-Cl | H | H | H | H | para | β-D-Glu-NHAc |
| 121 | 576 | CO | 2-$CH_3$ | H | H | H | H | para | β-D-Glu-NHAc |
| 122 | 583 | CO | 2-$CH_3$ | H | H | 3-$CH_3$ | H | para | β-D-Glu-NHAc |
| 123 | 589 | CHOH | 4-$NO_2$ | H | H | H | H | para | β-D-Glu-NHAc |
| 124 | 590 | CO | 4-Cl | H | H | H | H | para | β-D-Glu |
| 125 | 591 | CO | 2-OH | H | H | H | H | para | β-D-Xyl |
| 126 | 602 | CO | H | H | H | H | H | para | β-D-Glu |
| 127 | 605 | CO | 4-$CH_3$ | H | H | H | H | para | β-D-Glu |
| 128 | 618 | CHOH | 4-$CH_3$ | H | H | H | H | para | β-D-Glu-NHAc |
| 129 | 619 | CHOH | 4-$NO_2$ | H | H | H | H | para | β-D-Glu |
| 130 | 620 | CO | N | H | H | H | H | para | β-D-Glu-NHAc |
| 131 | 621 | CHOH | 4Cl | H | H | H | H | para | β-D-Glu |
| 132 | 680 | CO | 3-$CF_3$ | H | H | H | H | méta | β-D-Xyl |
| 133 | 651 | CHOH | 4$CH_3$ | H | H | H | H | para | β-D-Glu |
| 134 | 653 | CHOH | H | H | H | H | H | para | β-D-Glu |
| 135 | 679 | CO | 4-$CH_3$ | H | H | 4-$CH_3$ | H | méta | β-D-Xyl |
| 136 | 678 | CHOH | 4-$NO_2$ | H | H | H | H | ortho | β-D-Xyl |
| 137 | – | CO | 4-Cl | H | H | H | H | para | β-D-Gal |
| 138 | – | CHOH | 4-$NH_2$ | H | H | H | H | para | β-D-Gal |
| 139 | – | CO | 4-$NO_2$ | H | H | H | H | para | α-L-Rham |
| 140 | – | CO | H | H | H | 2-$CH_3$ | H | para | β-D-Xyl |
| 141 | – | CO | 4-$NO_2$ | H | H | H | H | ortho | $(OAc)_3$-β-D-Xyl |

Notes:
[a] mélange des deux diastéréoisomères
[b] diastéréoisomère dextrogyre
[c] diastéréoisomère lévogyre
[d] Les exemples 106 et 107 sont des diastéréoisomères (voir pouvoirs rotatoires dans Tableau II)

Tableau II
Caracteristiques physiques

| Exemple | No de code | Point de fusion (°C) | pouvoir rotatoire | | |
|---|---|---|---|---|---|
| | | | $\alpha_D^{20°C}$ | Concentration | Solvant |
| 1 | 163 | 200 | −26,6 | 0,6 | MeOH |
| 2 | 459 | 135 | −30 | 0,15 | MeOH |

Tableau II (suite)

| Exemple | No de code | Point de fusion (°C) | pouvoir rotatoire $\alpha_D^{20°C}$ | Concen-tration | Solvant |
|---|---|---|---|---|---|
| 3 | 171 | 174 | −27,04 | 1,054 | MeOH |
| 4 | 433 | 182 | −23,8 | 0,84 | MeOH |
| 5 | 456 | 90 | −21 | 0,7 | MeOH |
| 6 | 511 | 130 | −23 | 0,5 | MeOH |
| 8 | 193 | 200 | −26,5 | 0,49 | MeOH |
| 9 | 466 | 150 | −26 | 0,3 | MeOH |
| 10 | 676 | 205 | −47 | 0,5 | MeOH |
| 11 | 227 | 160 | −25,4 | 0,61 | MeOH |
| 12 | 229 | 162 | −26,7 | 0,6 | MeOH |
| 13 | 465 | 100 | −26 | 0,35 | MeOH |
| 14 | 435 | 125 | −21 | 0,5 | MeOH |
| 15 | – | 126 | −6,8 | 0,5 | MeOH |
| 16 | 476 | 154 | −30 | 0,5 | MeOH |
| 17 | 486 | 180 | −26 | 0,5 | MeOH |
| 18 | 262 | 140 | −20 | 0,7 | AcOEt |
| 19 | 228 | 196 | −54,2 | 0,6 | MeOH |
| 20 | 264 | 220 | −39,34 | 0,61 | Pyridine |
| 21 | 243 | 132 | +17,25 | 0,58 | MeOH |
| 22 | 560 | 188 | +32 | 0,5 | MeOH |
| 23 | 487 | 185 | +13 | 0,25 | MeOH |
| 25 | 241 | 100 | −83,3 | 0,63 | MeOH |
| 26 | 242 | 265 | −9,4 | 0,6 | Pyridine |
| 27 | 357 | 222 | −12,8 | 0,7 | Pyridine |
| 29 | 457 | 200 | −27 | 0,2 | MeOH |
| 31 | 431 | 172 | −21,5 | 0,72 | MeOH |
| 32 | 462 | 130 | −24 | 0,5 | MeOH |
| 33 | 510 | 170 | −22 | 0,5 | MeOH |
| 37 | 397 | 220 | +17 | 0,2 | MeOH |
| 41 | 236 | 150 | −33,3 | 0,9 | $ClCH_2CH_2Cl$ |
| 42 | 260 | 143 | – | – | – |
| 43 | 172 | 240 | −8,8 | 0,91 | Pyridine |
| 44 | 207 | 206 | +12,5 | 0,6 | MeOH |
| 45 | 430 | 140 | −30 | 0,5 | MeOH |
| 46 | 650 | 108 | −29,6 | 0,5 | MeOH |
| 48 | 427 | 80 | −25,5 | 0,7 | MeOH |
| 49 | 432 | 154 | −28,6 | 0,77 | MeOH |
| 52 | 434 | 168 | −32,5 | 0,8 | MeOH |
| 53 | 428 | 120 | −38 | 0,5 | MeOH |
| 54 | 429 | 140 | −27 | 0,7 | MeOH |
| 56 | – | 129,5 | – | – | – |
| 57 | – | 164 | – | – | – |
| 60 | 677 | 187 | −68 | 0,6 | acétone |
| 66 | 289 | 144 | −22,13 | 0,61 | MeOH |
| 67 | 358 | 200 (avec dé-composition) | −2,1 | 2,6 | $H_2O$ |
| 68 | 416 | > 265 | +11 | 1 | $H_2O$ |
| 69 | 488 | 115 | −22 | 0,52 | MeOH |
| 70 | 490 | 165 | −24 | 0,5 | MeOH |
| 71 | 497 | 220 | −33 | 0,5 | MeOH |
| 72 | 498 | – | −40 | 0,2 | MeOH |
| 73 | 499 | 153 | −45 | 0,5 | MeOH |
| 74 | 500 | 140 | −20 | 0,5 | MeOH |
| 75 | 501 | 150 | −12 | 0,5 | MeOH |
| 76 | 502 | 138 | −22 | 0,5 | MeOH |
| 77 | 503 | 170 | −17 | 0,5 | MeOH |
| 78 | 518 | 145 | −25 | 0,5 | MeOH |
| 79 | 519 | 114 | −16 | 0,5 | MeOH |
| 80 | 520 | 145 | −26 | 0,5 | MeOH |

Tableau II (suite)

| Exemple | No de code | Point de fusion (°C) | pouvoir rotatoire | | |
|---|---|---|---|---|---|
| | | | $\alpha_D^{20°C}$ | Concentration | Solvant |
| 81 | 521 | 144 | −21 | 0,5 | MeOH |
| 82 | 522 | 126 | −26 | 0,5 | MeOH |
| 83 | 523 | 148 | −28 | 0,5 | MeOH |
| 84 | 525 | 162 | −48 | 0,5 | MeOH |
| 85 | 477 | 135 | −25 | 0,5 | MeOH |
| 86 | 512 | 152 | −16,7 | 0,54 | MeOH |
| 87 | 513 | 125 | −15 | 0,5 | MeOH |
| 88 | 514 | 210 | −23 | 0,5 | MeOH |
| 89 | 515 | 210 | −26 | 0,5 | MeOH |
| 90 | 516 | 206 | −24 | 0,5 | MeOH |
| 91 | 517 | 185 | −16 | 0,5 | MeOH |
| 92 | 526 | 190 | −26 | 0,5 | MeOH |
| 93 | 527 | 168 | −20 | 0,5 | MeOH |
| 94 | 528 | 84 | −37 | 0,5 | MeOH |
| 95 | 529 | 190 | −23 | 0,5 | MeOH |
| 96 | 559 | 80 | −25 | 0,5 | MeOH |
| 97 | 265 | 142 | −17 | 0,5 | MeOH |
| 98 | 390 | 162 | +21 | 0,48 | MeOH |
| 99 | 391 | 158 | −50 | 0,48 | MeOH |
| 100 | 411 | 218 | +13 | 0,5 | MeOH |
| 101 | 555 | 198 | −45 | 0,5 | MeOH |
| 102 | 551 | 190 | +13 | 0,5 | MeOH |
| 103 | 550 | 102 | −33 | 0,5 | MeOH |
| 104 | 549 | 164 | −46 | 0,5 | $CHCl_3$ |
| 105 | 558 | 110 | −9,5 | 0,42 | MeOH |
| 106 | 557 | 50 | −24 | 0,41 | MeOH |
| 107 | 556 | 50 | −11 | 0,45 | MeOH |
| 108 | 541 | 140 | −27 | 0,5 | MeOH |
| 109 | 561 | 240 | −64 | 0,50 | $CHCl_3$ |
| 110 | 562 | 218 | – | – | – |
| 111 | 563 | 212 | +25 | 0,20 | EtOH |
| 112 | 564 | 220 | −12,7 | 0,55 | $CHCl_3$ |
| 113 | 565 | 130 | −8 | 0,50 | $CHCl_3$ |
| 114 | 506 | 150 | +40 | 0,50 | $CHCl_3$ |
| 115 | 662 | 172 | – | – | – |
| 116 | 568 | 152 | – | – | – |
| 117 | 570 | 180 | −2,3 | 0,44 | MeOH |
| 118 | 573 | 238 | +20 | 0,40 | MeOH |
| 119 | 574 | 268 | – | – | – |
| 120 | 575 | 226 | +20 | 0,40 | MeOH |
| 121 | 576 | 230 | +22 | 0,50 | MeOH |
| 122 | 583 | 238 | +4 | 0,50 | MeOH |
| 123 | 589 | 215 | +20 | 0,50 | MeOH |
| 124 | 590 | 152 | – | – | – |
| 125 | 591 | 180 | −60 | 0,40 | MeOH |
| 126 | 602 | 163 | −54 | 0,5 | MeOH |
| 127 | 605 | 110 | −55 | 0,48 | MeOH |
| 128 | 618 | 210 | +72 | 0,2 | $EtOH–H_2O$ (4:1) v/v |
| 129 | 619 | 140 | −66 | 0,5 | MeOH |
| 130 | 620 | 250 | +20 | 0,5 | MeOH |
| 131 | 621 | 130 | −64 | 0,5 | MeOH |
| 132 | – | 135 | −66 | 0,5 | MeOH |
| 133 | 651 | 84 | −26,4 | 0,72 | MeOH |
| 134 | 653 | 90 | −43,8 | 0,48 | MeOH |
| 135 | 679 | 125 | −66 | 0,6 | MeOH |
| 136 | 678 | 207 | – | – | – |
| 137 | – | 202 | – | – | – |

Tableau II (suite)

| Exemple | No de code | Point de fusion (°C) | pouvoir rotatoire $\alpha_D^{20°C}$ | Concentration | Solvant |
|---|---|---|---|---|---|
| 138 | – | Décomposition à 60°C | – | – | – |
| 139 | – | 94 | | | |
| 140 | – | 110 | −61 | 0,3 | MeOH |
| 141 | – | 142 | – | – | – |

Tableau III
Toxicité aigëu chez la souris par voie I.P.

| Exemple | No de code | DL-0; DL-50 mg/kg IP |
|---|---|---|
| 1 | 163 | DL-0>800 |
| 2 | 459 | DL-0>800 |
| 3 | 171 | DL-0>800 |
| 4 | 433 | DL-0>800 |
| 5 | 456 | DL-0>800 |
| 6 | 511 | DL-50=600 |
| 7 | 554 | DL-0>800 |
| 8 | 193 | DL-0>800 |
| 11 | 227 | DL-0>800 |
| 12 | 229 | DL-0>800 |
| 13 | 465 | DL-50=170 |
| 14 | 435 | DL-50=740 |
| 16 | 476 | DL-0>800 |
| 17 | 486 | DL-50=1600 |
| 18 | 262 | DL-50>1000 |
| 19 | 228 | DL-0>800 |
| 20 | 264 | DL-0>800 |
| 21 | 243 | DL-50=600 |
| 22 | 560 | DL-50=500 |
| 23 | 487 | DL-50=750 |
| 25 | 241 | DL-50=330 |
| 26 | 242 | DL-50=700 |
| 27 | 357 | DL-0>800 |
| 28 | 572 | DL-0>800 |
| 29 | 457 | DL-0>800 |
| 31 | 431 | DL-0>800 |
| 32 | 462 | DL-0>800 |
| 33 | 510 | DL-0>800 |
| 37 | 397 | DL-50=600 |
| 41 | 236 | DL-0>800 |
| 42 | 260 | DL-0>800 |
| 43 | 172 | DL-0>800 |
| 44 | 207 | DL-0>800 |
| 45 | 430 | DL-0>800 |
| 48 | 427 | DL-50=220 |
| 49 | 432 | DL-0>800 |
| 52 | 434 | DL-0>800 |
| 53 | 428 | DL-50=550 |
| 54 | 429 | DL-0>800 |
| 65 | 222 | DL-0>800 |
| 67 | 358 | DL-0>800 |
| 68 | 416 | DL-0>800 |
| 69 | 488 | DL-50=900 |
| 70 | 490 | DL-0>800 |
| 71 | 497 | DL-0>800 |
| 72 | 498 | DL-50=650 |

Tableau III (suite)

| | | |
|---|---|---|
| 73 | 499 | DL-0>800 |
| 74 | 500 | DL-0>800 |
| 75 | 501 | DL-0>800 |
| 76 | 502 | DL-0>800 |
| 77 | 503 | DL-0>800 |
| 78 | 518 | DL-0>800 |
| 79 | 519 | DL-0>800 |
| 80 | 520 | DL-0>800 |
| 81 | 521 | DL-0>800 |
| 82 | 522 | DL-0>800 |
| 83 | 523 | DL-0>800 |
| 84 | 525 | DL-0>800 |
| 85 | 477 | DL-50=550 |
| 86 | 512 | DL-50=350 |
| 87 | 513 | DL-50=600 |
| 88 | 514 | DL-0>800 |
| 89 | 515 | DL-0>800 |
| 90 | 516 | DL-0>800 |
| 91 | 517 | DL-0>800 |
| 92 | 526 | DL-50=1400 |
| 93 | 527 | DL-0>800 |
| 94 | 528 | DL-50=330 |
| 95 | 529 | DL-0>800 |
| 97 | 265 | DL-50>1000 |
| 100 | 411 | DL-0>800 |
| 101 | 555 | DL-0>800 |
| 102 | 551 | DL-0>800 |
| 103 | 550 | DL-0>800 |
| 104 | 549 | DL-0>800 |
| 105 | 558 | DL-50=450 |
| 106 | 557 | DL-50=500 |
| 107 | 556 | DL-50=550 |
| 108 | 541 | DL-0>800 |
| 109 | 561 | DL-0>800 |
| 110 | 562 | DL-0>800 |
| 111 | 563 | DL-50=750 |
| 112 | 564 | DL-0>800 |
| 113 | 565 | DL-50=630 |
| 114 | 566 | DL-0>800 |
| 116 | 568 | DL-0>800 |
| 117 | 570 | DL-50=1000 |
| 118 | 573 | DL-0>800 |
| 119 | 574 | DL-0>800 |
| 120 | 575 | DL-0>800 |
| 121 | 576 | DL-0>800 |
| 122 | 583 | DL-0>800 |
| 123 | 589 | DL-0>800 |
| 124 | 590 | DL-0>800 |
| 125 | 591 | DL-50=1700 |
| 126 | 602 | DL-0>800 |
| 127 | 605 | DL-50=700 |

Tableau III (suite)

| Exemple | No de code | DL-0; DL-50 mg/kg IP |
|---|---|---|
| 128 | 618 | DL-0>800 |
| 129 | 619 | DL-0>800 |
| 130 | 620 | DL-0>800 |
| 131 | 621 | DL-50=730 |

Tableau IV
Activité antiulcéreuse

| Exemple | No de code | Index Ulcère |
|---|---|---|
| 1 | 163 | 0,56 |
| 2 | 459 | 0,84 |
| 4 | 433 | 1,04 |
| 6 | 511 | 0,45 |
| 8 | 193 | 0,25 |
| 11 | 227 | 0 (200 mg/kg P.O.) |
| 12 | 229 | 0,26 |
| 13 | 465 | 0,46 |
| 16 | 476 | 0,73 |
| 17 | 486 | 0,69 |
| 19 | 228 | 0,74 |
| 20 | 264 | 0,62 |
| 21 | 243 | 0,16 |
| 22 | 560 | 1,46 |
| 23 | 487 | 0,52 |
| 25 | 241 | 0,54 |
| 26 | 242 | 0,60 |
| 27 | 357 | 0,42 |
| 31 | 431 | 1,04 |
| 32 | 462 | 0,54 |
| 33 | 510 | 0,46 |
| 37 | 397 | 1,02 |
| 41 | 236 | 0,40 |
| 43 | 172 | 0,52 |
| 44 | 207 | 0,385 |
| 45 | 430 | 1,11 |
| 48 | 427 | 0,75 |
| 49 | 432 | 1,21 |
| 53 | 428 | 0,64 |
| 54 | 429 | 0,57 |
| 67 | 358 | 0,81 |
| 69 | 488 | 0,78 |
| 70 | 490 | 0,56 |
| 71 | 497 | 0,98 |
| 72 | 498 | 0,47 |
| 73 | 499 | 0,52 |
| 74 | 500 | 0,91 |
| 75 | 501 | 0,32 |
| 76 | 502 | 0,38 |
| 80 | 520 | 1,00 |
| 81 | 521 | 0,64 |
| 86 | 512 | 0,50 |
| 87 | 513 | 0,35 |
| 88 | 514 | 0,39 |
| 89 | 515 | 0,88 |
| 90 | 516 | 0,57 |
| 91 | 517 | 0,72 |
| 95 | 529 | 0,52 |

Tableau IV (suite)

| | | |
|---|---|---|
| 97 | 265 | 0,52 |
| 100 | 411 | 0,35 |
| 101 | 555 | 0,56 |
| 102 | 551 | 0,49 |
| 103 | 550 | 0,65 |
| 104 | 549 | 0,84 |
| 108 | 541 | 0,30 |
| 109 | 561 | 0,65 |
| 110 | 562 | 0,71 |
| 111 | 563 | 0,42 |
| 112 | 564 | 0,68 |
| 116 | 568 | 0,44 |
| 117 | 570 | 0,59 |
| 118 | 573 | 0,53 |
| 119 | 574 | 0,47 |
| 121 | 576 | 0,39 |
| 122 | 583 | 0,91 |

Tableau V
Activité antiagrégante des plaquettes sanguines

| Exemple | No de code | Inhibition d'agrégation plaquettaire[a] |
|---|---|---|
| 1 | 163 | + + |
| 8 | 193 | + + |
| 12 | 229 | + |
| 14 | 435 | + + (75 mg/kg IP) |
| 21 | 243 | + + |
| 54 | 429 | + + |
| 67 | 358 | + |
| 79 | 519 | + + |
| 94 | 529 | + (33 mg/kg IP) |
| 97 | 265 | + + |
| 102 | 551 | + + |
| 104 | 549 | + + |
| 105 | 558 | + (45 mg/kg IP) |
| 113 | 565 | + + (63 mg/kg IP) |
| 114 | 566 | + |
| 118 | 573 | + |

Note
[a] le pourcentage d'inhibition de l'agrégation plaquettaire correspond à la notation suivante
+ inhibition de 25 à 39%
+ + inhibition supérieure ou égale à 40%

Tableau VI
Activité antithrombotique veineuse

| Exemple | No de code | Diminution thrombus veineux[a] |
|---|---|---|
| 2 | 459 | + |
| 3 | 171 | + |
| 4 | 433 | + |
| 5 | 456 | + + |
| 6 | 511 | + |
| 7 | 554 | + + |
| 8 | 193 | + + |
| 14 | 435 | + + |
| 17 | 486 | + |
| 18 | 262 | + |

Tableau VI (suite)

| Exemple | No de code | Diminution thrombus veineux[a] |
|---|---|---|
| 23 | 487 | + |
| 29 | 457 | + |
| 45 | 430 | + |
| 54 | 429 | + |
| 67 | 358 | + |
| 69 | 488 | + |
| 70 | 490 | + |
| 71 | 497 | + |
| 75 | 501 | + |
| 76 | 502 | + |
| 77 | 503 | + |
| 81 | 521 | + |
| 85 | 477 | + + |
| 86 | 512 | + |
| 87 | 513 | + + |
| 88 | 514 | + + |
| 89 | 515 | + + |
| 91 | 517 | + |
| 92 | 526 | + |
| 97 | 265 | + + |
| 98 | 390 | + + |
| 99 | 391 | + |
| 101 | 555 | + + |
| 102 | 551 | + |
| 106 | 557 | + + (50 mg/kg IP) |
| 108 | 541 | + |
| 119 | 574 | + + |
| 123 | 589 | + |
| 125 | 591 | + |
| 130 | 620 | + |
| 131 | 621 | + |

Note
[a] notation:
+ inhibition de 30 à 49%
+ + inhibition supérieure ou égale à 50%

Tableau VII
Activité anti-hypoxique

| Exemple | No de code | Survie[a] |
|---|---|---|
| 1 | 163 | + + + |
| 3 | 171 | + + + |
| 8 | 193 | + + + |
| 11 | 227 | + + |
| 12 | 229 | + + |
| 13 | 465 | + + |
| 16 | 476 | + |
| 19 | 228 | + + |
| 26 | 242 | + |
| 28 | 572 | + + |
| 41 | 236 | + + + |
| 43 | 172 | + + + |
| 44 | 207 | + + + |
| 76 | 502 | + + |
| 83 | 523 | + + |
| 85 | 477 | + |

Tableau VII (suite)

| Exemple | No de code | Survie[a] |
|---|---|---|
| 91 | 517 | + + + |
| 94 | 528 | + |
| 100 | 411 | + + |
| 101 | 555 | + |
| 102 | 551 | + + + |
| 103 | 550 | + + |
| 104 | 549 | + + |
| 108 | 541 | + |
| 109 | 561 | + + |
| 110 | 562 | + + |
| 111 | 563 | + + |
| 112 | 564 | + + |
| 113 | 565 | + + + |
| 114 | 566 | + + |
| 116 | 568 | + |
| 117 | 570 | + + |
| 118 | 573 | + + |
| 119 | 574 | + + + |
| 120 | 575 | + + + |
| 121 | 576 | + |
| 122 | 583 | + |
| 123 | 589 | + + |
| 124 | 590 | + |
| 125 | 591 | + |
| 126 | 602 | + |
| 127 | 605 | + + |
| 128 | 618 | + + |
| 130 | 620 | + + |
| 131 | 621 | + + |

Note
[a] le pourcentage de survie à l'agression hypoxique est donné selon la notation:
+ de 30 à 49%
++ de 50 à 79%
+++ supérieur ou égal à 80%

**Revendications pour les Etats contractants: BE, CH, DE, FR, GB, IT, LI, LU, NL, SE**

1. Composé nouveau appartenant à la famille des benzoyl- et α-hydroxybenzyl-phényl-osides, caractérisé en ce qu'il est choisi parmi l'ensemble constitué par

(i) les composés répondant à la formule générale

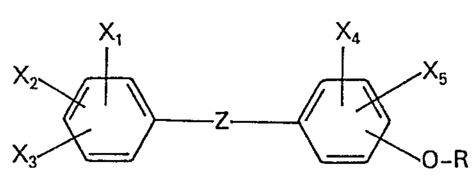

(I)

dans laquelle:

- Z représente >CO ou >CHOH;
- $X_2$, $X_3$, $X_4$ et $X_5$, identiques ou différents, représentent chacun un atome d'hydrogène, un atome d'halogène, un groupe alkyle en $C_1$–$C_4$, un groupe

alkyle en $C_1$–$C_4$ substitué par un ou plusieurs atomes d'halogène, un groupe OH, un groupe alkoxy en $C_1$–$C_4$, un groupe alkoxy en $C_1$–$C_4$ substitué par un ou plusieurs atomes d'halogène, un groupe nitro, un groupe cyano, un groupe thiocyano, un groupe isothiocyano, un groupe NR′R″ (où R′ et R″, identiques ou différents, représentent chacun l'atome d'hydrogène ou un groupe alkyle en $C_1$–$C_4$);

– $X_1$ représente un atome d'hydrogène, un atome d'halogène, un groupe alkyle en $C_1$–$C_4$, un groupe alkyle en $C_1$–$C_4$ substitué par un ou plusieurs atomes d'halogène, un groupe OH, un groupe alkoxy en $C_1$–$C_4$, un groupe alkoxy en $C_1$–$C_4$ substitué par un ou plusieurs atomes d'halogène, un groupe nitro, un groupe cyano, un groupe thiocyano, un groupe isothiocyano, un groupe NR′R″ (où R′ et R″, identiques ou différents, représentent chacun l'atome d'hydrogène ou un groupe alkyle en $C_1$–$C_4$), un groupe $-NH-CS-O-CH_3$ ou un groupe $-O-C(CH_3)_2CO_2-R'''$ (où R‴ est un groupe alkyle en $C_1$–$C_4$);

– R représente un radical ose choisi parmi l'ensemble constitué par

a) le radical α-L-rhamnosyle,

b) les radicaux monosaccharides non-hydrolysables, et

c) les radicaux monosaccharides non-hydrolysables où la fonction hydroxyle de l'atome de carbone en position 2 est remplacée par une fonction amine,

les fonctions hydroxyle et amine du groupe R étant susceptibles d'être acétylées, méthylées, benzylées ou sulfatées; et

(ii) leurs diastéréoisomères quand Z est CHOH.

2. Composé selon la revendication 1, caractérisé en ce que $X_1$, $X_2$, $X_3$, $X_4$ et $X_5$, identiques ou différents, représentent chacun H, Cl, Br, $CH_3$, OH, $OCH_3$, $NO_2$, $NH_2$ $N(CH_3)_2$, NCS, $X_1$ représentant également en position para, par rapport ou groupe Z, un groupe $-OC(CH_3)_2CO_2-CH(CH_3)_2$ ou un groupe $-NH-CS-OCH_3$ quand $X_2 = X_3 = X_4 = X_5 = H$; et R représente un radical choisi parmi l'ensemble constitué par les radicaux α-L-rhamnosyle, β-D-glucosyle, β-D-xylosyle, β-D-galactosyle et β-D-glucosaminyle, dans lesquels, le cas échéant, l'atome d'hydrogène des groupes OH peut être remplacé par un reste $COCH_3$, $CH_3$, $CH_2C_6H_5$, $SO_3NH_4$, $SO_3Na$,$SO_3K$, et la fonction amine du radical β-D-glucosaminyle peut être substituée par un groupe $COCH_3$.

3. [4-(4-Nitrobenzoyl)-phényl]-2,3,4-tri-(O-acétyl)-β-D-xylopyranoside.

4. [4-(4-Nitrobenzoyl)-phényl]-β-D-xylopyranoside.

5. [3-(4-Trifluorméthylbenzoyl)-phényl]-β-D-xylopyranoside.

6. [4-(4-Chlorobenzoyl)-phényl]-3,4,6-tri-(ammoniumsulfate)-2-N-acétyl-β-D-glucosaminide.

7. [4-(4-Nitrobenzoyl)-phényl]-2-N-acétyl-β-D-glucosaminide.

8. [4-(4-Nitro-α-hydroxybenzyl)-phényl]-β-D-xylopyranoside et ses diastéréoisomères.

9. [4-(4-Nitro-α-hydroxybenzyl)-phényl]-2,3,4-tri-(O-acétyl)-β-D-xylopyranoside.

10. Composition thérapeutique, caractérisée en ce qu'elle renferme, en association avec un excipient physiologiquement acceptable, au moins un composé selon l'une quelconque des revendications 1 à 9.

11. Procédé de préparation d'un composé de formule (I) selon la revendication 1 dans lequel Z est CO ou CHOH, caractérisé en ce que:

(i) on fait réagir un dérivé de phényl-phénol de formule:

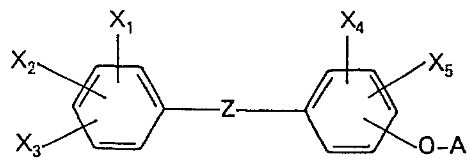

(II)

(où Z, $X_1$, $X_2$, $X_3$, $X_4$ et $X_5$ sont définis comme indiqué ci-dessus, et A représente H, Na ou K) avec un dérivé ose choisi parmi l'ensemble constitué par les halogénoacétyloses et les acétyloses, dans lesquels le groupe acétylose est un groupe R acétylé tel que défini dans la revendication 1, dans un solvant inerte, à raison de 1 mole de (II) pour 1,1 à 1,2 mole de dérivé ose; et

(ii) si nécessaire, on procède à une réaction de désacétylation.

12. Procédé selon la revendication 11, caractérisé en ce que l'on fait réagir au stade (i) 1 mole de dérivé de phényl-phénol (II) (où A est Na ou K) avec 1,1 à 1,2 mole d'un halogénoacétylose de formule

Hal-Ro (III)

(où Hal est F, Cl, Br ou I, et Ro représente un reste ose R acétylé) dans un solvant inerte polaire ou apolaire choisi parmi l'ensemble constitué par a) les diméthylformamide, dioxanne, tétrahydrofuranne, méthanol, éthanol, acétonitrile, nitrométhane, diméthylsulfoxyde et leurs mélanges, et b) les mélanges DMF-$CH_2Cl_2$, DMF-$CHCl_3$ et DMF-$ClCH_2CH_2Cl$.

13. Procédé selon la revendication 12, caractérisé en ce que la réaction de (II) avec (III) est effectuée à une température comprise entre 0°C et la température de reflux du milieu réactionnel, pendant 10 à 40 minutes.

14. Procédé selon la revendication 11, caractérisé en ce que la réaction de désacétylation du stade (ii) est effectuée par chauffage à reflux du dérivé acétylé dans le méthanol en présence d'un alcoolate métallique choisi parmi le méthylate de magnésium et méthylate de sodium.

15. Procédé de préparation d'un dérivé de formule (I) selon la revendication 1, où Z est CHOH, à partir d'un composé de formule I où Z est CO, ledit procédé étant caractérisé en ce que l'on soumet le dérivé de formule I où Z est CO à une réaction de réduction dans un solvant inerte, au moyen d'un agent réducteur choisi parmi $LiAlH_4$ et $KBH_4$, à une température comprise entre 0°C et 50°C.

16. Procédé pour la résolution d'un mélange

de diastéréoisomères, de formule (I), selon la revendication 1, où Z est CHOH, par recristallisation fractionnée, ledit procédé étant caractérisé en ce que a) on dissout le mélange des diastéréoisomères dans le mélange méthanol-eau (1:1) v/v et procède à des recristallisations successives jusqu'à pouvoir rotatoire constant, pour recueillir le diastéréoisomère dextrogyre, puis b) on reprend les filtrats des recristallisations du stade a) dans le mélange méthanol-eau (1:2) v/v et procède à des recristallisations successives jusqu'à pouvoir rotatoire constant, pour recueillir le diastéréoisomère lévogyre.

**Revendications pour l'état contractant: AT**

1. Procédé pour préparer un composé appartenant à la famille des benzoyl- et α-hydroxybenzyl-phényl-oside répondant à la formule générale

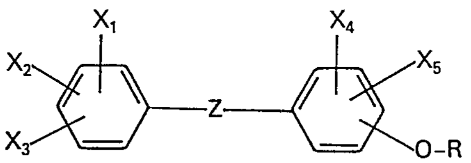

(I)

dans laquelle:
- Z représente > CO ou > CHOH;
- $X_2$, $X_3$, $X_4$ et $X_5$, identiques ou différents, représentent chacun un atome d'hydrogène, un atome d'halogène, un groupe alkyle en $C_1$-$C_4$, un groupe alkyle en $C_1$-$C_4$ substitué par un ou plusieurs atomes d'halogène, un groupe OH, un groupe alkoxy en $C_1$-$C_4$ un groupe alkoxy en $C_1$-$C_4$ substitué par un ou plusieurs atomes d'halogène, un groupe nitro, un groupe cyano, un groupe thiocyano, un groupe isothiocyano, un groupe NR'R'' (où R' et R'', identiques ou différents, représentent chacun l'atome d'hydrogène ou un groupe alkyle en $C_1$-$C_4$);
- $X_1$ représente un atome d'hydrogène, un atome d'halogène, un groupe alkyle en $C_1$-$C_4$, un groupe alkyle en $C_1$-$C_4$ substitué par un ou plusieurs atomes d'halogène, un groupe OH, un groupe alkoxy en $C_1$-$C_4$ un groupe alkoxy en $C_1$-$C_4$ substiué par un ou plusieurs atomes d'halogène, un groupe nitro, un groupe cyano, un groupe thiocyano, un groupe isothiocyano, un groupe NR'R'' (où R' et R'', identiques ou différents, représentent chacun l'atome d'hydrogène ou un groupe alkyle en $C_1$-$C_4$), un groupe $-NH-CS-O-CH_3$ ou un groupe $-O-C(CH_3)_2CO_2-R'''$ (où R''' est un groupe alkyle en $C_1$-$C_4$),
- R représente un radical ose choisi parmi l'ensemble constitué par
a) le radical α-L-rhamnosyle,
b) les radicaux monosaccharides non-hydrolysables, et
c) les radicaux monosaccharides non-hydrolysables où la fonction hydroxyle de l'atome de carbone en position 2 est remplacée par une fonction amine,
les fonctions hydroxyle et amine du groupe R étant susceptibles d'être acétylées, méthylées, benzylées ou sulfatées; et leurs diastéréoisomères quand Z est CHOH, ledit procédé étant caractérisé en ce que

(i) on fait réagir un dérivé de phényl-phénol de formule:

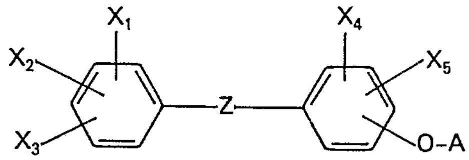

(II)

(où Z, $X_1$, $X_2$, $X_3$, $X_4$ et $X_5$ sont définis comme indiqué ci-dessus, et A représente H, Nn ou K) avec un dérivé ose choisi parmi l'ensemble constitué par les halogénoacétyloses et les acétyloses, dans lesquels le groupe acétylose est un groupe R acétylé tel que défini ci-dessus, dans un solvant inerte, à raison de 1 mole de (II) pour 1,1 à 1,2 mole de dérivé ose; et

(ii) si nécessaire, on procède à un réaction de désacétylation.

2. Procédé selon la revendication 1, caractérisé en ce que l'on fait réagir au stade (i) 1 mole de dérivé de phényl-phénol (II) (où A est Na ou K) avec 1,1 à 1,2 mole d'un halogénoacétylose de formule

Hal-Ro (III)

(où Hal est F, Cl, Br ou I, et Ro représente un reste ose R acétylé) dans un solvant inerte polaire ou apolaire choisi parmi l'ensemble constitué par a) les diméthylformamide, dioxanne, tétrahydrofuranne, méthanol, éthanol, acétonitrile, nitrométhane, diméthylsulfoxyde et leurs mélanges, et b) les mélanges DMF-$CH_2Cl_2$, DMF-$CHCl_3$ et DMF-$ClCH_2CH_2Cl$.

3. Procédé selon la revendication 2, caractérisé en ce que la réaction de (II) avec (III) est effectuée à une température comprise entre 0°C et la température de reflux du milieu réactionnel, pendant 10 à 40 minutes.

4. Procédé selon la revendication 1, caractérisé en ce que la réaction de désacétylation du stade (ii) est effectuée par chauffage à reflux du dérivé acètylose dans le méthanol en présence d'un alcoolate métallique choisi parmi le méthylate de magnésium et méthylate de sodium.

5. Procédé de préparation d'un dérivé de formule (I) selon la revendication 1, où Z est CHOH, à partir d'un composé de formule I où Z est CO, ledit procédé étant caractérisé en ce que l'on soumet le dérivé de formule I où Z est CO à une réaction de réduction dans un solvant inerte, au moyen d'un agent réducteur choisi parmi $LiAlH_4$ et $KBH_4$, à une température comprise entre 0°C et 50°C.

6. Procédé pour la résolution d'un mélange de diastéréoisomères, de formule (I), selon la revendication 1, où Z est CHOH, par recristallisation fractionnée, ledit procédé étant caractérisé en ce que a) on dissout le mélange des diastéréoisomères dans le mélange méthanol-eau (1:1) v/v et procède à des rescristallisations successives jusqu'à pouvoir rotatoire constant, pour recueillir le diastéréoisomère dextrogyre, puis b) on reprend les filtrats des recristallisations du stade a) dans le mélange méthanol-eau (1:2) v/v et procède à des recristallisations successives jusqu'à pouvoir rotatoire constant, pour recueillir le diastéréoisomère lévogyre.

**Patentansprüche für die Vertragsstaaten: BE, CH, DE, FR, GB, IT, LI, LU, NL, SE.**

1. Verbindung aus der Familie der Benzoyl- und α-Hydroxybenzyl-phenyl-oside, dadurch gekennzeichnet, dass die aus der Gruppierung die aus

(i) den Verbindungen der allgemeinen Formel

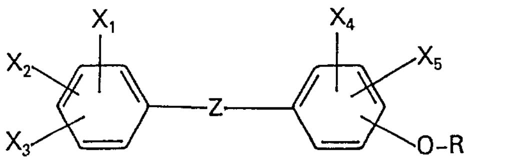

(I)

worin

- Z > O oder > CHOH bedeutet;
- $X_2$, $X_3$, $X_4$ und $X_5$, gleich oder verschieden, jeweils ein Wasserstoffatom, ein Halogenatom, eine $C_1$–$C_4$-Alkylgruppe, eine durch ein oder mehrere Halogenatome substituierte $C_1$–$C_4$-Alkylgruppe, eine OH-Gruppe, eine $C_1$–$C_4$-Alkoxygruppe, eine durch ein oder mehrere Halogenatome substituierte $C_1$–$C_4$-Alkoxygruppe, eine Nitrogruppe, eine Cyanogruppe, eine Thiocyanogruppe, eine Isothiocyanogruppe oder eine NR'R"-Gruppe (in der R' und R", gleich oder verschieden, jeweils ein Wasserstoffatom oder eine $C_1$–$C_4$-Alkylgruppe darstellen) bedeuten;
- $X_1$, ein Wasserstoffatom, ein Halogenatom, eine $C_1$–$C_4$-Alkylgruppe, eine durch ein oder mehrere Halogenatome substituierte $C_1$–$C_4$-Alkylgruppe, eine OH-Gruppe, eine $C_1$–$C_4$-Alkoxygruppe, eine durch ein oder mehrere Halogenatome substituierte $C_1$–$C_4$-Alkoxygruppe, eine Nitrogruppe, eine Cyanogruppe, eine Thiocyanogruppe, eine Isothiocyanogruppe, eine NR'R"-Gruppe (in der R' und R", gleich oder verschieden, jeweils ein Wasserstoffatom oder eine $C_1$–$C_4$-Alkylgruppe darstellen), eine -NH-CS-O-$CH_3$-Gruppe oder eine -O-$C(CH_3)_2CO_2$R'''-Gruppe (in der R''' eine $C_1$–$C_4$-Alkylgruppe darstellt) bedeutet;
- R ein aus

a) α-L-Rhamnosyl,

b) Radikalen von unhydrolysierbaren Monosacchariden, und

b) Radikalen von unhydrolysierbaren Monosacchariden in welchen die Hydroxyl-Funktion in 2-Stellung durch eine Amin-Funktion ersetzt ist, gewähltes Osyl-Radikal, worin die Hydroxyl- und Amin-Funktionen gegebenenfalls acetyliert, methyliert oder sulfatiert sein können, bedeutet; und,

(ii) ihren Diastereoisomeren, wenn Z die CHOH-Gruppe darstellt;

besteht, gewählt wird.

2. Verbindung gemäss Anspruch 1, dadurch gekennzeichnet, dass $X_1$, $X_2$, $X_3$, $X_4$ et $X_5$, gleich oder verschieden, jeweils H, Cl, Br, $CH_3$, $CF_3$, OH, $OCH_3$, $NO_2$, $NH_2$ oder NCS bedeuten, wobei $X_1$ ebenfalls in para-Stellung zur Z-Gruppe eine $-OC(CH_3)_2CO_2CH(CH_3)_2$-Gruppe oder eine -NH-CS-$OCH_3$-Gruppe, wenn $X_2$, $X_3$, $X_4$ und $X_5$ H sind, bedeuten kann; und R ein Osyl-Radikal, das aus α-L-Rhamnosyl, β-Glukosyl, β-D-Xylosyl, β-D-Galaktosyl und β-D-Glukosaminyl, wobei gegebenenfalls die Wasserstoffatome der OH-Gruppen des Osyl-Radikals durch einen $COCH_3$, $CH_3$, $CH_2C_6H_5$, $SO_3NH_4$, $SO_3Na$ oder $SO_3K$ Rest ersetzt sein können und die Amin-Funktion des β-D-Glukosaminyl-Restes durch eine $COCH_3$-Gruppe substituiert sein kann, gewählt wird.

3. [4-(4-Nitrobenzoyl)-phenyl]-2,3,4-tri-(O-acetyl)-β-D-xylopyranosid.

4. [4-(4-Nitrobenzoyl)-phenyl]-β-D-xylopyranosid.

5. [3-(4-Trifluormethylbenzoyl)-phenyl]-β-D-xylopyranosid.

6. [4-(4-Chlorobenzoyl-phenyl]-3,4,6-tri-(ammoniumsulfat)-2-N-acetyl-β-D-glukosaminid.

7. [4-(4-Nitrobenzoyl)-phenyl]-2-N-acetyl-β-D-glukosaminid.

8. [4-(4-Nitro-α-hydroxybenzyl)-phenyl]-β-D-xylopyranosid und seine Diastereoisomeren.

9. [4-(4-Nitro-α-hydroxybenzyl)-phenyl]-2,3,4-tri(O-acetyl)-β-D-xylopyranosid.

10. Therapeutische Zusammensetzung, dadurch gekennzeichnet, dass sie, in Kombination mit einem physiologisch verträglichen Hilfsstoff, mindestens eine Verbindung nach Ansprüchen 1 bis 9 enthält.

11. Verfahren zur Herstellung einer Verbindung der Formel I nach Anspruch 1, worin Z CO oder CHOH ist, dadurch gekennzeichnet, dass man

(i) ein Derivat des Phenyl-phenols der Formel

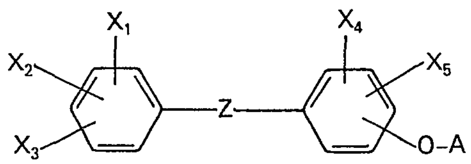

(II)

(worin Z, $X_1$, $X_2$, $X_3$, $X_4$ und $X_5$ wie oben definiert sind, und A Wasserstoff, Na oder K bedeutet) mit einem Ose-Derivat, das aus der Gruppierung der Halogenoacetylosen und Acetylosen (worin die Acetylose-Gruppe eine acetylierte R-Gruppe, die wie oben im Anspruch 1 definiert ist, darstellt) gewählt wird, in einem inerten Lösungsmittel umsetzt, wobei man mit einem Verhältnis von 1 Mol Verbindung II zu 1,1 bis 1,2 Mol Ose-Derivate arbeitet; und

(ii) wann erforderlich, eine Desacetylierung durchführt.

12. Verfahren gemäss Anspruch 11, dadurch gekennzeichnet, dass man 1 Mol des Phenyl-phenols der Formel II (worin A Na oder K ist), mit 1,1 bis 1,2 Mol einer Halogenoacetylose-Verbindung der Formel

$$Hal-R_o \qquad III$$

(worin Hal F, Cl, Br und J ist, und $R_0$ eine acetylierte R-Osylgruppe) umsetzt, wobei man in einem aus

a) Dimethylformamid, Dioxan, Tetrahydrofuran, Methanol, Äthanol, Acetonitril, Nitromethan, Dimethylsulfoxid und ihren Mischungen, und

b) DMF-$CH_2Cl_2$, DMF-$CHCl_3$ und DMF-$ClCH_2CH_2Cl$ Mischungen,

gewählten inerten polaren oder unpolaren Lösungsmittel arbeitet.

13. Verfahren gemäss Anspruch 12, dadurch gekennzeichnet, dass die Reaktion von II mit III bei einer Temperatur zwischen 0°C und der Rückflusstemperatur der Reaktionsmischung während 10 bis 40 Minuten durchgeführt wird.

14. Verfahren gemäss Anspruch 11, dadurch gekennzeichnet, dass die Desacetylierung der Stufe (ii) mittels Erhitzung des Acetyl-Derivats unter Rückfluss im Methanol in Anwesenheit eines aus Magnesiummethylat und Natriummethylat gewählten Metall-Alkoholats durchgeführt wird.

15. Verfahren zur Herstellung einer Verbindung der Formel I nach Anspruch 1, worin Z CHOH darstellt, ausgehend von einer Verbindung der Formel I, in der Z CO ist, dadurch gekennzeichnet, dass man die Ausgangsverbindung, in der Z CO ist, mit einem Reduktionsmittel behandelt, wobei die Reduktion in einem inerten Lösungsmittel bei einer Temperatur zwischen 0°C und 50°C mit einem aus $LiAlH_4$ und $KBH_4$ gewählten Reduktionsmittel durchgeführt wird.

16. Verfahren zur Trennung einer Mischung von Diastereoisomeren einer Verbindung der Formel I nach Anspruch 1, in der Z CHOH ist, durch fraktionierte Umkristallisation, dadurch gekennzeichnet, dass man

a) die Mischung der Diastereoisomeren in einer Methanol-Wasser-Mischung (1:1, v/v,) löst und die schrittweise Umkristallisation bis zu einem konstanten Drehvermögen vornimmt, um das rechtsdrehende Diastereoisomer zu sammeln; und

b) die Umkristallisationsfiltrate der Stufe a) wieder in einer Methanol-Wasser-Mischung (1:2, v/v) löst und die schrittweise Umkristallisation bis zu einem konstanten Drehvermögen vornimmt, um das linksdrehende Diastereoisomer zu sammeln.

**Patentansprüche für den Vertragsstaat: AT**

1. Verfahren zur Herstellung einer Verbindung der Familie der Benzoyl- und α-Hydroxylbenzylphenyl-oside der allgemeinen Formel

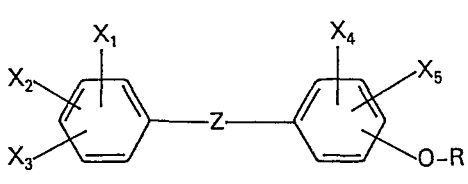

(I)

worin

- Z > CO oder > CHOH bedeutet;

- $X_2$, $X_3$, $X_4$ und $X_5$, gleich oder verschieden, jeweils ein Wasserstoffatom, ein Halogenatom, eine $C_1$–$C_4$-Alkylgruppe, eine durch ein oder mehrere Halogenatome substituierte $C_1$–$C_4$-Alkylgruppe, eine OH-Gruppe, eine $C_1$–$C_4$-Alkoxygruppe, eine durch ein oder mehrere Halogenatome substituierte $C_1$–$C_4$-Alkoxygruppe, eine Nitrogruppe, eine Cyanogruppe, eine Thiocyanogruppe, eine Isothiocyanogruppe, oder eine NR′R″-Gruppe, (in der R′ und R″, gleich oder verschieden, jeweils ein Wasserstoffatom oder eine $C_1$–$C_4$-Alkylgruppe darstellen) bedeuten;

- $X_1$ ein Wasserstoffatom, ein Halogenatom, eine $C_1$–$C_4$-Alkylgruppe, eine durch ein oder mehrere Halogenatome substituierte $C_1$–$C_4$-Alkylgruppe, eine OH-Gruppe, eine $C_1$–$C_4$-Alkoxygruppe, eine durch ein oder mehrere Halogenatome substituierte $C_1$–$C_4$-Alkoxygruppe, eine Nitrogruppe, eine Cyanogruppe, eine Thiocyanogruppe, eine Isothiocyanogruppe, eine NR′R″-Gruppe (in der R′ und R″, gleich oder verschieden, jeweils ein Wasserstoffatom oder eine $C_1$–$C_4$ Alkylgruppe darstellen); eine $-NH-CS-O-CH_3$-Gruppe oder eine $-O-C(CH_3)_2CO_2R'''$-Gruppe (in der R‴ eine $C_1$–$C_4$-Alkylgruppe darstellt) bedeutet;

- R ein aus

a) α-L-Rhamnosyl,

b) Radikalen von unhydrolysierbaren Monosacchariden,

und

b) Radikalen von unhydrolysierbaren Monosacchariden in welchen die Hydroxyl-Funktion in 2-Stellung durch eine Amin-Funktion ersetzt ist,

gewähltes Osyl-Radikal, worin die Hydroxyl- und Amin-Funktionen gegebenenfalls acetyliert, methyliert oder sulfatiert sein können bedeutet; und ihren Diastereoisomeren, wenn Z die CHOH-Gruppe darstellt; dadurch gekennzeichnet, dass man

(i) ein Derivat des Phenyl-phenols der Formel

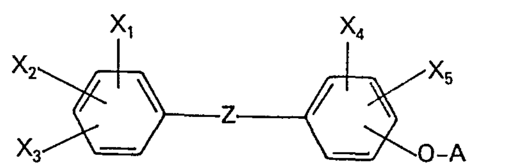

(II)

(worin Z, $X_1$, $X_2$, $X_3$, $X_4$ et $X_5$ wie oben definiert sind, und A Wasserstoff, Na oder K bedeutet) mit einem Ose-Derivat, das aus der Gruppierung der Halogenoacetylosen und Acetylosen (worin die Acetylose-Gruppe eine acetylierte R-Gruppe, die wie oben definiert ist, darstellt) gewählt wird, in einem inerten Lösungsmittel umsetzt, wobei man mit einem Verhältnis von 1 Mol Verbindung II zu 1,1 bis 1,2 Mol Ose-Derivat arbeitet; und

(ii) wann erforderlich, eine Desacetylierung durchführt.

Verfahren gemäss Anspruch 1, dadurch gekennzeichnet, dass man 1 Mol des Phenyl-phenols der Formel II (worin A Na oder K ist) mit 1,1 bis 1,2 Mol einer Halogenoacetylose-Verbindung der Formel

Hal-$R_0$ III

(worin Hal F, Cl, Br und J ist, und $R_0$ eine acetylierte R-Osylgruppe) umsetzt, wobei man in einem aus

a) Dimethylformamid, Dioxan, Tetrahydrofuran, Methanol, Äthanol, Acetonitril, Nitromethan, Dimethylsulfoxid und ihren Mischungen und

b) den DMF-$CH_2Cl_2$, DMF-$CHCl_3$ und DMF-$ClCH_2CH_2Cl$ Mischungen,

gewählten inerten polaren oder unpolaren Lösungsmittel arbeitet.

3. Verfahren gemäss Anspruch 2, dadurch gekennzeichnet, dass die Reaktion von II mit III bei einer Temperatur zwischen 0°C und der Rückflusstemperatur der Reaktionsmischung während 10 bis 40 Minuten durchgeführt wird.

4. Verfahren gemäss Anspruch 1, dadurch gekennzeichnet, dass die Desacetylierung der Stufe (ii) mittels Erhitzung des Acetyl-Derivats unter Rückfluss im Methanol in Anwesenheit eines aus Magnesiummethylat und Natriummethylat gewählten Metall-Alkoholats durchgeführt wird.

5. Verfahren zur Herstellung einer Verbindung der Formel I nach Anspruch 1, worin Z CHOH darstellt, ausgehend von einer Verbindung der Formel I, in der Z CO ist, dadurch gekennzeichnet, dass man die Ausgangsverbindung, in der Z CO ist, mit einem Reduktionsmittel behandelt, wobei die Reduktion in einem inerten Lösungsmittel bei einer Temperatur zwischen 0°C und 50°C mit einem aus $LiAlH_4$ und $KBH_4$ gewählten Reduktionsmittel durchgeführt wird.

6. Verfahren zur Trennung einer Mischung von Diastereoisomeren einer Verbindung der Formel I nach Anspruch 1, in der Z CHOH ist, durch fraktionierte Umkristallisation, dadurch gekennzeichnet, dass man

a) die Mischung der Diastereoisomeren in einer Methanol-Wasser-Mischung (1:1, v/v) löst und die schrittweise Umkristallisation bis zu einem konstanten Drehvermögen, um das rechtsdrehende Diastereoisomer zu sammeln, vornimmt, und

b) die Umkristallisationsfiltrate der Stufe a) wieder in einer Methanol-Wasser-Mischung (1:2, v/v) löst und die schrittweise Umkristallisation bis zu einem konstanten Drehvermögen, um das linksdrehende Diastereoisomer zu sammeln, vornimmt.

**Claims for the contracting states: BE, CH, DE, FR, GB, IT, LI, LU, NL, SE**

1. A compound belonging to the family of benzoyl- and α-hydroxybenzyl-phenyl-osides, characterised in that it is selected from the group consisting of:

(i) compounds of the general formula:

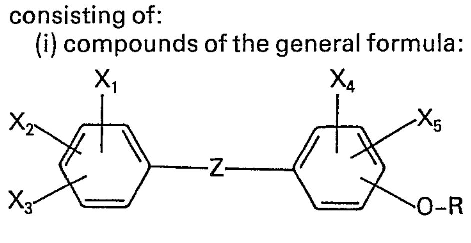

(I)

wherein

- Z represents > CO or > CHOH,
- $X_2$, $X_3$, $X_4$ and $X_5$, which may be identical or different, represent a hydrogen atom, a halogen atom, a $C_1$–$C_4$-alkyl group, a $C_1$–$C_4$ alkyl group substituted by one or more halogen atoms, an OH group, a $C_1$–$C_4$-alkoxy group, a $C_1$–$C_4$-alkoxy group substituted by one or more halogen atoms, a nitro group, a cyano group, a thiocyano group, an isothiocyano group, a NR′R″ group (wherein R′ and R″, which may be identical or different, represent a hydrogen atom or a $C_1$–$C_4$-alkyl group);
- $X_1$ represents a hydrogen atom, a halogen atom, a $C_1$–$C_4$ alkyl-group, a $C_1$–$C_4$-alkyl group substituted by one or more halogen atoms, an OH group, a $C_1$–$C_4$-alkoxy group, a $C_1$–$C_4$-alkoxy group substituted by one or more halogen atoms, a nitro group, a cyano group, a thiocyano group, an isothiocyano group, a NR′R″ group (wherein R′ and R″, which may be identical or different, represent a hydrogen atom or a $C_1$–$C_4$-alkyl group), a $-NH-CS-O-CH_3$ group or a $-O-C(CH_3)_2CO_2-R'''$ group (wherein R‴ is a $C_1$–$C_4$-alkyl group)
- R represents an ose radical selected from the group consisting of

a) the α-L-rhamnosyl radical,

b) the radicals of non-hydrolysable monosaccharides,

and

c) the radicals of non-hydrolysable monosaccharides wherein the hydroxyl function of the carbon atom in 2-position is replaced by an amine function,

the hydroxyl and amine functions of the R group being able to be acetylated, methylated or sulphated; and

(ii) diastereoisomers thereof when Z is CHOH.

2. A compound according to claim 1, characterised in that $X_1$, $X_2$, $X_3$, $X_4$ and $X_5$, which may be identical or different, represent H, Cl, Br, $CH_3$, $CF_3$, OH, $OCH_3$, $NO_2$, $NH_2$, $N(CH_3)_2$, NCS, $X_1$ also representing in para position with respect to the Z group an $-OC(CH_3)_2CO_2-CH(CH_3)_2$ or an $-NH-CS-OCH_3$ group when $X_2 = X_3 = X_4 = X_5 = H$; and R represents an ose radical selected from the group consisting of α-L-rhamnosyl, β-D-glucosyl, β-D-xylosyl, β-D-galactosyl and β-D-glucosaminyl, wherein the hydrogen atoms of the OH groups may be replaced by a $COCH_3$, $CH_3$, $CH_2C_6H_5$, $SO_3Na$ or $SO_3K$ group, and the amine function of the β-D-glucosaminyl radical may be substituted by a $COCH_3$ group.

3. [4-(4-Nitrobenzoyl)-phenyl]-2,3,4-tri-(O-acetyl)-β-D-xylopyranoside.

4. [4-(4-Nitrobenzoyl)-phenyl]-β-D-xylopyranoside.

5. [3-(4-Trifluoromethylbenzoyl)-phenyl]-β-D-xylopyranoside.

6. [4-(4-Chlorobenzoyl)-phenyl]-3,4,6-tri-(ammoniumsulphate)-2-N-acetyl-β-glucosaminide.

7. [4-(4-Nitrobenzoyl)-phenyl]-2-N-acetyl-β-D-glucosaminide.

8. [4-(4-Nitro-α-hydroxybenzyl)-phenyl]-β-D-xylopyranoside and its diastereoisomers.

9. [4-(4-Nitro-α-hydroxybenzyl)-phenyl]-2,3,4-tri-(O-acetyl)-β-D-xylopyranoside.

10. A therapeutical composition, characterised in that it contains, in association with a physiologically acceptable excipent, at least a compound according to any one of claims 1 to 9.

11. A method for preparing a compound of formula (I) according to claim 1, in which Z is CO or CHOH, characterised in that:

(i) a phenyl-phenol derivative of the formula:

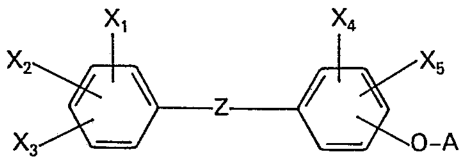

(II)

(wherein Z, $X_1$, $X_2$, $X_3$, $X_4$ et $X_5$ are defined as indicated hereinabove, and A represents H, Na or K) is reacted with an ose derivative selected from the group consisting of halogenoacetyloses and acetyloses inwhichtheacetylose group is an acetylated R group as defined in claim 1, in an inert solvent, at a rate of 1 mole of (II) for 1,1 to 1,2 mole of ose derivative; and,

(ii) if necessary, a deacetylation reaction is carried out.

12. A method according to claim 11, characterised in that 1 mole of phenyl-phenol derivative (II) (wherein A is Na or K) is reacted at step (i) with 1,1 to 1,2 mole of a halogenoacetylose of the formula

Hal-Ro (III)

(wherein Hal is F, Cl, Br or I, and Ro represents an acylated R radical) in an inert polar or apolar solvent selected from the group consisting of

a) dimethylformamide, dioxan, tetrahydrofuran, methanol, ethanol, acetonitrile, nitromethane, dimethylsulfoxide, and mixtures thereof, and

b) the DMF-$CH_2Cl_2$, DMF-$CHCl_3$, DMF-$ClCH_2CH_2Cl$ mixtures.

13. A method according to claim 12, characterised in that the deacetylation reaction of (II) with (III) is carried out at a temperature comprised between 0°C and the reflux temperature of the reaction medium, for 10 to 40 minutes.

14. A method according to claim 11, characterised in that the deacetylation reaction of step (ii) is carried out by heating the acetylated derivative to reflux in methanol in the presence of a metallic alcoholate selected from magnesium methylate and sodium methylate.

15. A method for preparing a derivative of formula I according to claim 1, wherein Z is CHOH, from a compound of formula I wherein Z is CO, said method being characterised in that the derivative of formula I where Z is CO is subjected to a reduction reaction in an inert solvent, by means of a reducing agent selected from $LiAlH_4$ and $KBH_4$, at a temperature comprised between 0°C and 50°C.

16. A method for the resolution of a mixture of diastereoisomers of formula I, according to claim 1 wherein Z is CHOH, by fractional recrystallisation, said method being characterised in that a) the mixture of the diastereoisomers is dissolved in a methanol-water (1:1) v/v mixture and successive recrystallisations are carried out up to constant rotatory power, to collect the dextrorotatory diastereoisomer, then b) the filtrates of the recrystallisations of step a) are taken up in a methanol-water (1:2) v/v mixture and successive recrystallisations are carried out up to constant rotatory power, to collect the laevorotatory diastereoisomer.

**Claims for the contracting state: AT**

1. A method for preparing a compound belonging to the family of benzoyl- and α-hydroxybenzyl-phenyl-osides of the general formula:

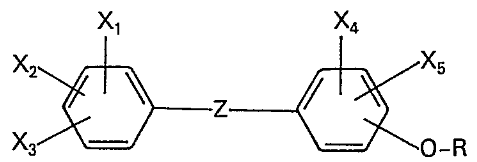

(I)

wherein

- Z represents > CO or > CHOH;
- $X_2$, $X_3$, $X_4$ and $X_5$, which may be identical or different, represent a hydrogen atom, a halogen atom, a $C_1$-$C_4$-alkyl group, a $C_1$-$C_4$-alkyl group substituted by one more halogen atoms, an OH group, a $C_1$-$C_4$-alkoxy group, a $C_1$-$C_4$-alkoxy group substituted by one or more halogen atoms, a nitro group, a cyano group, a thiocyano group, an isothiocyano group, a NR′R″ group (wherein R′ and R″, which may be identical or different, represent a hydrogen atom or a $C_1$-$C_4$-alkyl group)
- $X_1$ represents a hydrogen atom, a halogen atom, a $C_1$-$C_4$-alkyl group, a $C_1$-$C_4$-alkyl group substituted by one or more halogen atoms, an OH group, a $C_1$-$C_4$-alkoxy group, a $C_1$-$C_4$-alkoxy group substituted by one or more halogen atoms, a nitro group, a cyano group, a thiocyano group, an isothiocyano group, a NR′R″ group (wherein R′ and R″, which may be identical or different, represent a hydrogen atom or a $C_1$-$C_4$-alkyl group, a -NH-CS-O-$CH_3$ group or a -O-$C(CH_3)_2$-$CO_2R'''$ group (wherein R‴ is a $C_1$-$C_4$-alkyl group);
- R represents an ose radical selected from the group consisting of:

a) the α-L-rhamnosyl radical,

b) the radicals of non-hydrolysable monosaccharides,

and

c) the radicals of non-hydrolysable mono - saccharides wherein the hydroxyl function of the carbon atom in 2-position is replaced by an amine function,

the hydroxyl and amine functions of the R group

being able to be acetylated, methylated or sulphated; and diastereoisomers thereof when Z is OH; said method being characterised in that

(i) a phenyl-phenol derivative of the formula

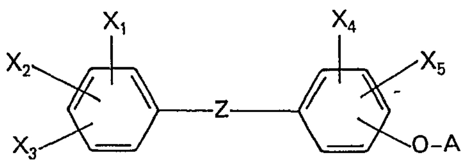

(II)

(where Z, $X_1$, $X_2$, $X_3$, $X_4$ and $X_5$ are defined as indicated hereinabove, and A represents H, Na or K) is reacted with an ose derivative selected from the group consisting of halogenoacetyloses and acetyloses in which the acetylose group is an acetylated R group as defined above, in an inert solvent, at a rate of 1 mole (II) for 1,1 to 1,2 mole of ose derivative; and,

(ii) if necessary, a deacetylation reaction is carried out.

2. A method according to claim 1, characterised in that 1 mole of phenyl-phenol derivative (II) (wherein A is Na or K) is reacted at step (i) with 1,1 to 1,2 mole of a halogenoacetylose of the formula

Hal-Ro (III)

(wherein Hal is F, Cl, Br or I, and Ro represents an acylated R radical) in an inert polar or apolar solvent selected from the group consisting of a) dimethylformamide, dioxan, tetrahydrofuran, methanol, ethanol, acetonitrile, nitromethane, dimethylsulfoxide, and mixtures thereof, and

b) the DMF-$CH_2Cl_2$, DMF-$CHCl_3$, DMF-$ClCH_2CH_2Cl$ mixtures.

3. A method according to claim 2, characterised in that the deacetylation reaction of (II) with (III) is carried out at a temperature comprised between 0°C and the reflux temperature of the reaction medium, for 10 to 40 minutes.

4. A method according to claim 1, characterised in that the deacetylation reaction of step (ii) is carried out by heating the acetylated derivative to reflux in methanol, in the presence of a metallic alcoholate selected from magnesium methylate, and sodium methylate.

5. A method for preparing a derivative of formula I according to claim 1, wherein Z is CHOH, from a compound of formula I wherein Z is CO, said method being characterised in that the derivative of formula I wherein Z is CO is subjected to a reduction reaction in an inert solvent, by means of a reducing agent selected from $LiAlH_4$ and $KBH_4$, at a temperature comprised between 0°C and 50°C.

6. A method for the resolution of a mixture of diastereoisomers of formula I, according to claim 1, wherein Z is CHOH, by fractional recrystallisation, said method being characterised in that a) the mixture of the diastereoisomers is dissolved in a methanol-water (1:1) v/v mixture and successive recrystallisations are carried out up to constant rotatory power, to collect the dextrorotatory diastereoisomer, then b) the filtrates of the recrystallisations of step a) are taken up in a methanol-water (1:2) v/v mixture and successive recrystallisations are carried out up to constant rotatory power, to collect the laevorotatory diastereoisomer.